# EUROPEAN PATENT APPLICATION

(11) **EP 4 464 697 A1**
(43) Date of publication of application: **20.11.2024**
(21) Application number: 23740461.1
(22) Date of filing: 12.01.2023
(51) Int. Cl.: C07D 307/78, A61K 31/7048

(54) **METHOD FOR PRODUCING INTERMEDIATE USEFUL FOR SYNETHESIS OF SGLT INHIBITOR**

(30) Priority: 14.01.2022 KR 20220005965
(71) Applicant: Daewoong Pharmaceutical Co., Ltd., Hwaseong-si, Gyeonggi-do 18623 (KR)
(72) Inventor: YOON, Hee Kyoon, Cheongju-si Chungcheongbuk-do 28774 (KR); YOON, Youn Jung, Yongin-si Gyeonggi-do 16899 (KR)
(74) Representative: Plasseraud IP
(86) International application number: PCT/KR2023/000549
(87) International publication number: WO 2023/136617

(57) **Abstract**

The present invention relates to a method for producing an intermediate useful for the synthesis of an SGLT inhibitor. According to the present invention, a compound of Chemical Formula 9, which is an important intermediate for a compound of Chemical Formula 1 as an SGTL inhibitor, may be obtained with high yield and high quality. A method for producing the SGLT inhibitor according to the present invention enables production without special facilities such as an ozone generator and thus is also much more economical.

## Description

### [Technical Field]

The present invention relates to a method for producing an intermediate useful for the synthesis of an SGLT inhibitor

### [Background Art]

A sodium-dependent glucose co-transporter (SGLT) allows the transport of Na⁺ along the concentration gradient simultaneously with the transport of glucose against the concentration gradient. In recent years, two important SGLT isoforms have been cloned and are known as SGLT1 and SGLT2. SGLT1 is located in the intestine, kidney, and heart and regulates cardiac glucose transport through its expression. SGLT1 is a high-affinity, low-capacity transporter, and is thus only partially responsible for renal glucose reabsorption. In contrast, SGLT2 is a low-affinity, high-capacity transporter located primarily in the apical domain of epithelial cells within the early proximal convoluted tubules. In the case of healthy individuals, more than 99% of the plasma glucose filtered through the renal glomeruli is reabsorbed, with less than 1% of the total filtered glucose excreted in urine. It is estimated that 90% of renal glucose reabsorption is catalyzed by SGLT2 and the remaining 10% is mediated by SGLT1 in the late proximal tubules. Genetic mutations in SGLT2 have no particular adverse effects on carbohydrate metabolism, but cause increased renal glucose secretion of approximately 140 g/day depending on the mutation. SGLT2 has become the target of therapeutic research because human mutation studies suggest that it is responsible for most renal glucose reabsorption.

US Patent Publication No. 2015/0152075 (Patent Document 1) discloses a compound containing a diphenylmethane residue having inhibitory activity against SGLT2 and a method for producing the same. This document discloses that the diphenylmethane derivative compound has an excellent inhibitory effect on human SGLT2 activity and is effective in treating diabetes by significantly reducing urinary glucose excretion in animals compared to dapagliflozin, which is a well-known SGLT2 inhibitor. Also, US Patent Publication No. 2014/0274918 discloses diphenylmethane derivatives effective as dual inhibitors for sodium-dependent glucose co-transporter 1 (SGLT1) and sodium-dependent glucose co-transporter 2 (SGLT2).

Example 172 of US Patent Publication No. 2015/0152075 (Patent Document 1) discloses a method for producing a diphenylmethane compound c28, which is useful as an SGLT inhibitor, in the same manner as Scheme 1 below.

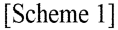

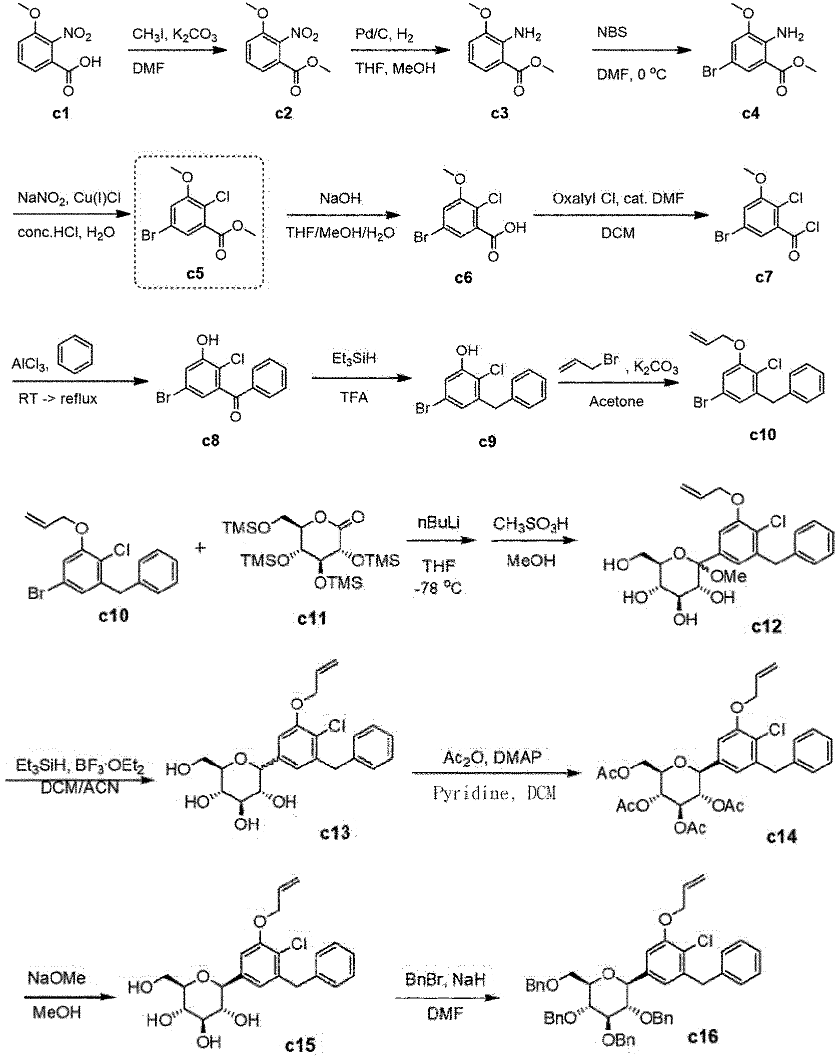

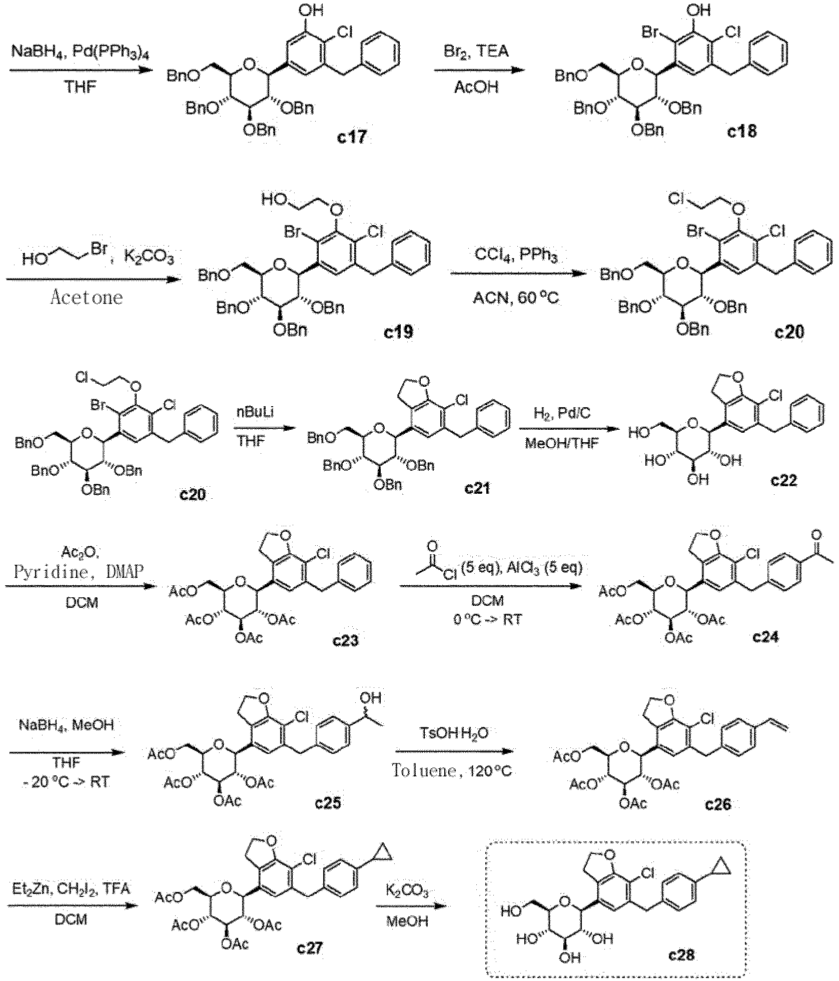

However, according to the conventional method for producing Compound c28, a linear synthesis method is adopted, such as forming a pentagonal ring from an aglycon group after coupling with a glucose group. In the case of such linear synthesis, the final yield is low due to the complicated route, and there also is the inconvenience of having to proceed with the synthesis again from the beginning when the synthesis of the substituent of the glucose group or the cyclopropylbenzyl group bonded to dihydrobenzofuran is performed incorrectly during the reaction or when it is desired to change to a different synthesis process. Also, because the process of synthesizing the cyclopropyl group of Compound c28 is also performed by cyclizing an olefin through the Simon-Smith reaction at the end of the route of synthesis, there is a problem in that the yield varies greatly depending on the state (purity, anhydrousness, and the like) of a reagent (diethyl zinc, a solvent), and the reaction concentration.

Meanwhile, Korea Patent Publication No. 2017-0142904 (Patent Document 2) discloses a method for producing a diphenylmethane derivative having inhibitory activity against SGLT2. This document discloses that the route of synthesis is simple, the yield may be enhanced, and the risk factors inherent in the linear route of synthesis may be reduced compared to the linear synthesis method disclosed in the above related-art document because the diphenylmethane derivatives are produced using a convergent synthesis method, which includes: individually synthesizing and coupling major groups, as shown in Schemes 2a and 2b below.

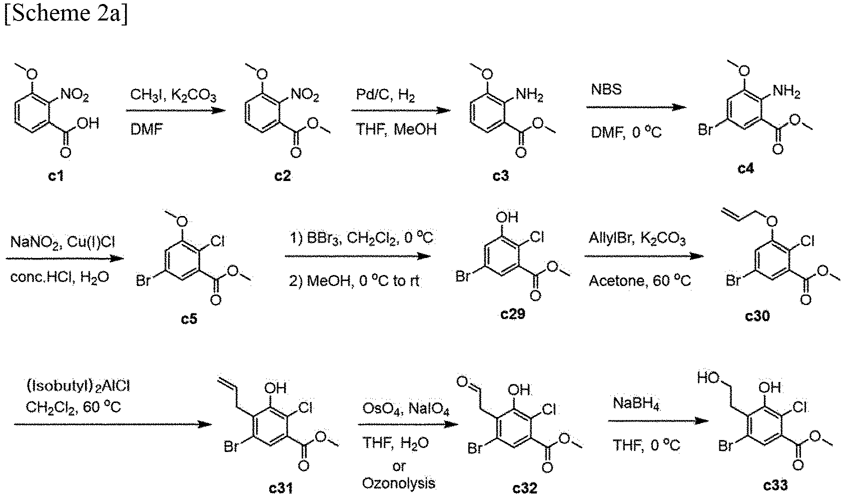

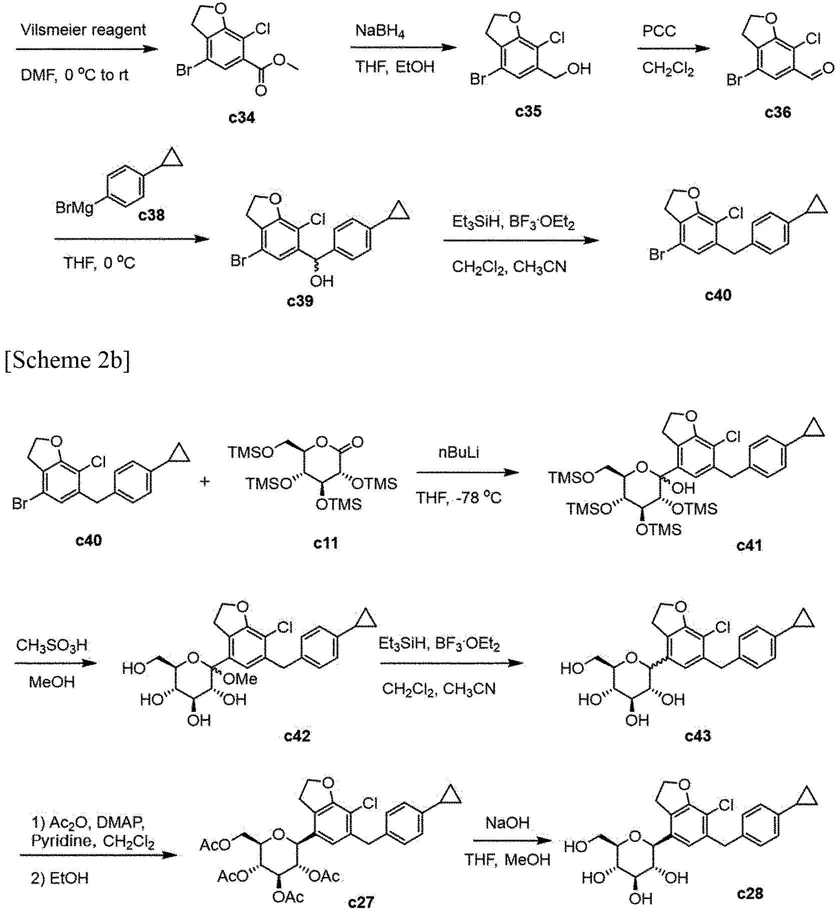

However, because 14 tedious steps are required to synthesize c40, which is an important intermediate for producing diphenylmethane compound c28 useful as the SGLT inhibitor (Scheme 2a), and special equipment such as an ozone generator is required when proceeding with c1 as the starting material, there are difficulties in terms of total yield and economic feasibility.

### [Related-Art Documents]

### [Patent Documents]

US Patent Publication No. 2015/0152075
Korea Patent Publication No. 2017-0142904

### [Disclosure]

### [Technical Problem]

Therefore, it is an object of the present invention to provide a novel method for producing a compound of Chemical Formula 9, which is an intermediate useful for the synthesis of an SGLT inhibitor.

### [Technical Solution]

The present inventors have developed a synthesis method with a short reaction step and high overall yield using more commercialized starting materials in synthesizing the compound of Chemical Formula 9, which is an important intermediate for a compound of the following Chemical Formula I. Therefore, the present invention has been completed based on the above findings.

The compound of Chemical Formula I, which is the final target compound and the active ingredient used as an SGLT inhibitor, is as follows: 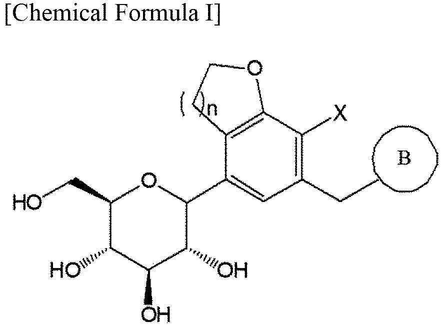 wherein:
n is 1 or 2,
X is a halogen (e.g., F, Cl, Br, or I), and
B is 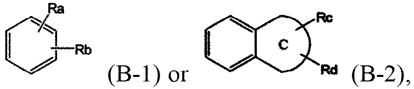
wherein Ra, Rb, Rc, and Rd are each independently hydrogen, a halogen, hydroxy, mercapto, cyano, nitro, amino, carboxy, oxo, a C1-7 alkyl, a C1-7 alkylthio, a C2-7 alkenyl, a C2-7 alkynyl, a C1-7 alkoxy, a C1-7 alkoxy-C1-7 alkyl, a C2-7 alkenyl-C1-7 alkyloxy, a C2-7 alkynyl-C1-7 alkyloxy, a C3-10 cycloalkyl, a C3-7 cycloalkylthio, a C5-10 cycloalkenyl, a C3-10 cycloalkyloxy, a C3-10 cycloalkyloxy-C1-7 alkoxy, a phenyl-C1-7 alkyl, a C1-7 alkylthio-phenyl, a phenyl-C1-7 alkoxy, a mono- or di-C1-7 alkylamino, a mono- or di-C1-7 alkylamino-C1-7 alkyl, a C1-7 alkanoyl, a C1-7 alkanoylamino, a C1-7 alkylcarbonyl, a C1-7 alkoxycarbonyl, carbamoyl, a mono- or di-C1-7 alkylcarbamoyl, a C1-7 alkylsulfonylamino, phenylsulfonylamino, a C1-7 alkylsulfinyl, a C6-14 arylsulfanyl, a C6-14 arylsulfonyl, a C6-14 aryl, a 5-13-membered heteroaryl, a 5-10-membered heterocycloalkyl, a 5-10-membered heterocycloalkyl-C1-7 alkyl, or a 5-10-membered heterocycloalkyl-C1-7 alkoxy;
ring C is a C3-10 cycloalkyl, a C5-10 cycloalkenyl, a C6-14 aryl, a 5-13-membered heteroaryl, or a 5-10-membered heterocycloalkyl;
the alkyl, the alkenyl, the alkynyl, and the alkoxy are each independently unsubstituted or have one or more substituents selected from the group consisting of a halogen, hydroxy, cyano, nitro, amino, mercapto, a C1-7 alkyl, and a C2-7 alkynyl;
the cycloalkyl, the cycloalkenyl, the aryl, the heteroaryl, and the heterocycloalkyl are each independently unsubstituted or have one or more substituents selected from the group consisting of a halogen, hydroxy, cyano, nitro, amino, mercapto, a C1-4 alkyl, and a C1-4 alkoxy; and
the heteroaryl and the heterocycloalkyl each independently contain one or more heteroatoms selected from the group consisting of N, S, and O.

According to one exemplary embodiment of the present invention, ring B-1 may be selected from the group consisting of the following: 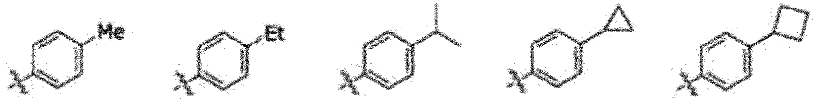 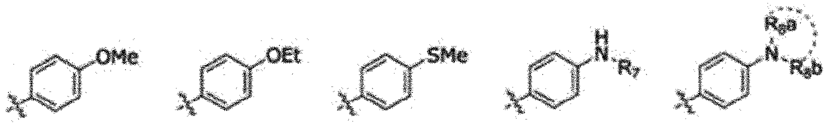 wherein R₇ is hydrogen or a C1-7 alkyl; R₈ₐ and R_{8b} are each independently a C1-7 alkyl, or are linked together to form a 5- to 10-membered heterocycloalkyl (containing one or more heteroatoms selected from the group consisting of N, S, and O).

According to another exemplary embodiment, ring B-2 may be selected from the group consisting of the following:

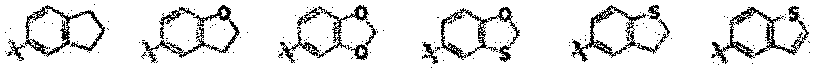

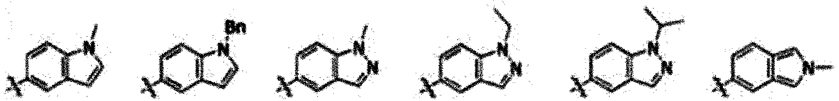

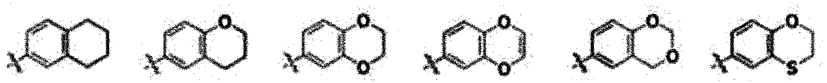

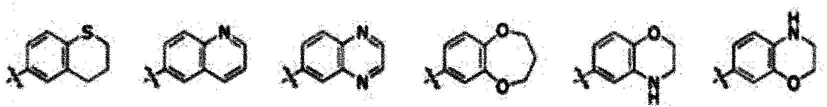

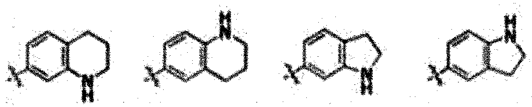

According to one preferred example of the compound of Chemical Formula I, n may be 1; X may be a halogen; and B may be phenyl unsubstituted or substituted with one or two substituents selected from the group consisting of a halogen, hydroxy, cyano, nitro, amino, mercapto, a C1-7 alkyl, a C3-10 cycloalkyl, and a C1-7 alkoxy.

Also, the compound of Chemical Formula I may be a compound in which the binding site between the diphenylmethane derivative and the heterocycloalkyl ring is in an α-form, a β-form, or a racemic form thereof.

For example, the compound of Chemical Formula I may be a compound of the following Chemical Formula Ia: 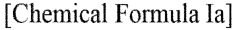 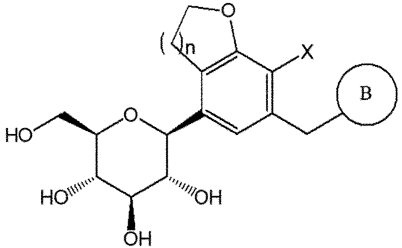 wherein B, n, and X are as defined above.

According to one aspect of the present invention, there is provided a method for producing a compound of the following Chemical Formula 9, which is an intermediate used to produce the diphenylmethane derivative of Chemical Formula I: 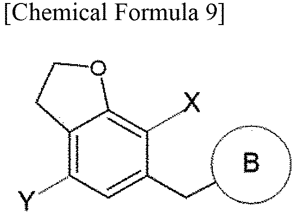 wherein:
X and Y are each independently a halogen, and
B is 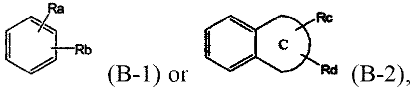
wherein Ra, Rb, Rc, and Rd are each independently hydrogen, a halogen, hydroxy, mercapto, cyano, nitro, amino, carboxy, oxo, a C1-7 alkyl, a C1-7 alkylthio, a C2-7 alkenyl, a C2-7 alkynyl, a C1-7 alkoxy, a C1-7 alkoxy-C1-7 alkyl, a C2-7 alkenyl-C1-7 alkyloxy, a C2-7 alkynyl-C1-7 alkyloxy, a C3-10 cycloalkyl, a C3-7 cycloalkylthio, a C5-10 cycloalkenyl, a C3-10 cycloalkyloxy, a C3-10 cycloalkyloxy-C1-7 alkoxy, a phenyl-C1-7 alkyl, a C1-7 alkylthio-phenyl, a phenyl-C1-7 alkoxy, a mono- or di-C1-7 alkylamino, a mono- or di-C1-7 alkylamino-C1-7 alkyl, a C1-7 alkanoyl, a C1-7 alkanoylamino, a C1-7 alkylcarbonyl, a C1-7 alkoxycarbonyl, carbamoyl, a mono- or di-C1-7 alkylcarbamoyl, a C1-7 alkylsulfonylamino, phenylsulfonylamino, a C1-7 alkylsulfinyl, a C6-14 arylsulfanyl, a C6-14 arylsulfonyl, a C6-14 aryl, a 5-13-membered heteroaryl, a 5-10-membered heterocycloalkyl, a 5-10-membered heterocycloalkyl-C1-7 alkyl, or a 5-10-membered heterocycloalkyl-C1-7 alkoxy;
ring C is a C3-10 cycloalkyl, a C5-10 cycloalkenyl, a C6-14 aryl, a 5-13-membered heteroaryl, or a 5-10-membered heterocycloalkyl;
the alkyl, the alkenyl, the alkynyl, and the alkoxy are each independently unsubstituted or have one or more substituents selected from the group consisting of a halogen, hydroxy, cyano, nitro, amino, mercapto, a C1-7 alkyl, and a C2-7 alkynyl;
the cycloalkyl, the cycloalkenyl, the aryl, the heteroaryl, and the heterocycloalkyl are each independently unsubstituted or have one or more substituents selected from the group consisting of a halogen, hydroxy, cyano, nitro, amino, mercapto, a C1-4 alkyl, and a C1-4 alkoxy; and
the heteroaryl and the heterocycloalkyl each independently contain one or more heteroatoms selected from the group consisting of N, S, and O.

According to another aspect of the present invention, there is provided a method for producing a compound of Chemical Formula 9, which includes the following steps:
obtaining a compound of Chemical Formula 6 from a compound of Chemical Formula 5; and
reacting a compound of Chemical Formula 7 with the compound of Chemical Formula 6 to obtain a compound of Chemical Formula 8: 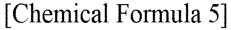 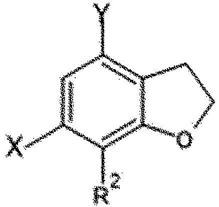 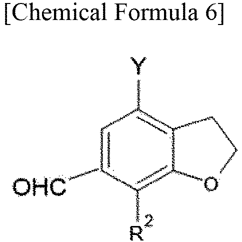 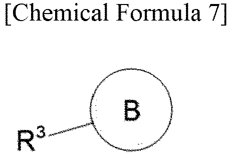 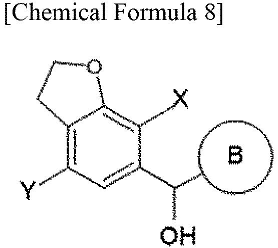 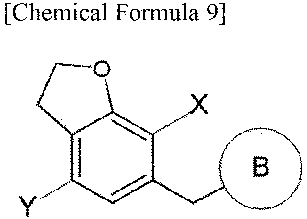 wherein:
R², R³, X, and Y are each independently a halogen, and
B is 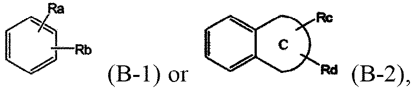
wherein Ra, Rb, Rc, and Rd are each independently hydrogen, a halogen, hydroxy, mercapto, cyano, nitro, amino, carboxy, oxo, a C1-7 alkyl, a C1-7 alkylthio, a C2-7 alkenyl, a C2-7 alkynyl, a C1-7 alkoxy, a C1-7 alkoxy-C1-7 alkyl, a C2-7 alkenyl-C1-7 alkyloxy, a C2-7 alkynyl-C1-7 alkyloxy, a C3-10 cycloalkyl, a C3-7 cycloalkylthio, a C5-10 cycloalkenyl, a C3-10 cycloalkyloxy, a C3-10 cycloalkyloxy-C1-7 alkoxy, a phenyl-C1-7 alkyl, a C1-7 alkylthio-phenyl, a phenyl-C1-7 alkoxy, a mono- or di-C1-7 alkylamino, a mono- or di-C1-7 alkylamino-C1-7 alkyl, a C1-7 alkanoyl, a C1-7 alkanoylamino, a C1-7 alkylcarbonyl, a C1-7 alkoxycarbonyl, carbamoyl, a mono- or di-C1-7 alkylcarbamoyl, a C1-7 alkylsulfonylamino, phenylsulfonylamino, a C1-7 alkylsulfinyl, a C6-14 arylsulfanyl, a C6-14 arylsulfonyl, a C6-14 aryl, a 5-13-membered heteroaryl, a 5-10-membered heterocycloalkyl, a 5-10-membered heterocycloalkyl-C1-7 alkyl, or a 5-10-membered heterocycloalkyl-C1-7 alkoxy;
ring C is a C3-10 cycloalkyl, a C5-10 cycloalkenyl, a C6-14 aryl, a 5-13-membered heteroaryl, or a 5-10-membered heterocycloalkyl;
the alkyl, the alkenyl, the alkynyl, and the alkoxy are each independently unsubstituted or have one or more substituents selected from the group consisting of a halogen, hydroxy, cyano, nitro, amino, mercapto, a C1-7 alkyl, and a C2-7 alkynyl;
the cycloalkyl, the cycloalkenyl, the aryl, the heteroaryl, and the heterocycloalkyl are each independently unsubstituted or have one or more substituents selected from the group consisting of a halogen, hydroxy, cyano, nitro, amino, mercapto, a C1-4 alkyl, and a C1-4 alkoxy; and
the heteroaryl and the heterocycloalkyl each independently contain one or more heteroatoms selected from the group consisting of N, S, and O.

In Korea Patent Publication No. 2017-0142904 (Patent Document 2), the process of producing the compound of Chemical Formula 9 consists of a total of 14 steps (Scheme 2a). Also, there was a problem that special equipment such as an ozone generator was needed when the process proceeds with c1 as the starting material. When there was no ozone reaction equipment, the reaction could be carried out using OsO₄/NaIO₄. In this case, when OsO₄/NaIO₄ was used, there was a problem that more impurities were generated as it is scaled up, making it impossible to apply it to production.

In order to solve the above problems of the related art, according to one embodiment of the present invention, commercially available 2,3-dihydrobenzofuran (a compound of the following Chemical Formula 1), 2,3-dihydrobenzofuran-7-carboxylic acid (a compound of the following Chemical Formula 2), or 2,3-dihydrobenzofuran-7-amine (a compound of the following Chemical Formula 3) (including salt forms) may be used as the starting material to obtain the compound of Chemical Formula 9.

According to one exemplary embodiment of the present invention, the compound of Chemical Formula 9 may be synthesized with a high yield of 39% by selective bromination and formylation through a total of 7 efficient processes using 2,3-dihydrobenzofuran (a compound of the following Chemical Formula 1) as the starting material when compared to the total yield of 26% in the existing method according to Korea Patent Publication No. 2017-0142904 (Patent Document 2), and production efficiency may also be maximized by reducing the number of synthesis steps to a total of 7 steps. In addition, the compound of Chemical Formula 9 may be produced in general facilities by eliminating the need for special equipment such as an ozone generator, and economic feasibility may also be greatly improved as it may be produced with high yield.

According to one exemplary embodiment, the method for producing a compound of Chemical Formula 9 according to the present invention may include the following steps 1 to 7 to produce the compound of Chemical Formula 9. In this case, because the starting material may be selected from the compound of Chemical Formula 1, the compound of Chemical Formula 2, the compound of Chemical Formula 3, or the compound of Chemical Formula 4, one or more of steps 1 to 4 may not be necessary:
obtaining a compound of Chemical Formula 2 from a compound of Chemical Formula 1 (step 1),
obtaining a compound of Chemical Formula 3 from the compound of Chemical Formula 2 (step 2),
obtaining a compound of Chemical Formula 4 from the compound of Chemical Formula 3 (step 3),
obtaining a compound of Chemical Formula 5 from the compound of Chemical Formula 4 (step 4),
obtaining a compound of Chemical Formula 6 from the compound of Chemical Formula 5 (step 5),
reacting a compound of Chemical Formula 7 with the compound of Chemical Formula 6 to obtain a compound of Chemical Formula 8 (step 6), and
obtaining the compound of Chemical Formula 9 from the compound of Chemical Formula 8 (step 7): 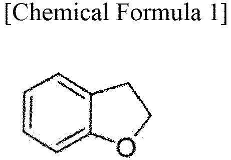 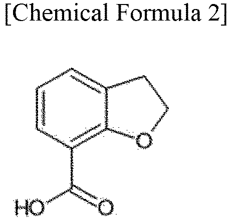 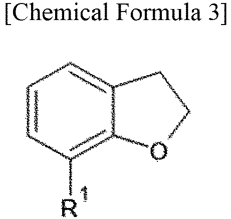 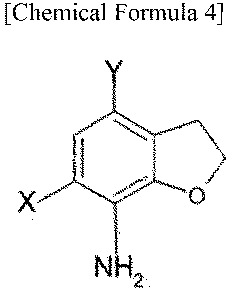 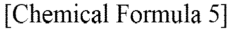 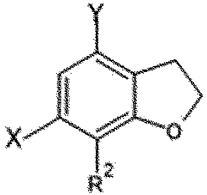 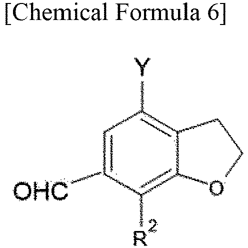 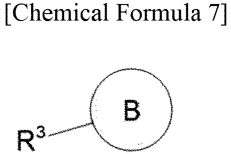 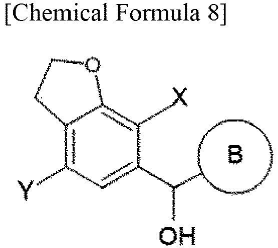 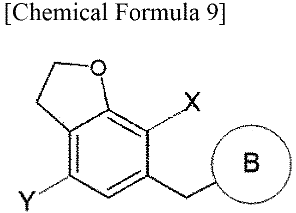
wherein:
   R¹ is NH₂,
   R², R³, X, and Y are each independently a halogen, and
   B is 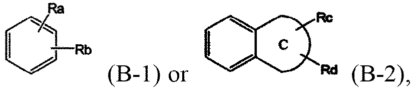
   wherein Ra, Rb, Rc, and Rd are each independently hydrogen, a halogen, hydroxy, mercapto, cyano, nitro, amino, carboxy, oxo, a C1-7 alkyl, a C1-7 alkylthio, a C2-7 alkenyl, a C2-7 alkynyl, a C1-7 alkoxy, a C1-7 alkoxy-C1-7 alkyl, a C2-7 alkenyl-C1-7 alkyloxy, a C2-7 alkynyl-C1-7 alkyloxy, a C3-10 cycloalkyl, a C3-7 cycloalkylthio, a C5-10 cycloalkenyl, a C3-10 cycloalkyloxy, a C3-10 cycloalkyloxy-C1-7 alkoxy, a phenyl-C1-7 alkyl, a C1-7 alkylthio-phenyl, a phenyl-C1-7 alkoxy, a mono- or di-C1-7 alkylamino, a mono- or di-C1-7 alkylamino-C1-7 alkyl, a C1-7 alkanoyl, a C1-7 alkanoylamino, a C1-7 alkylcarbonyl, a C1-7 alkoxycarbonyl, carbamoyl, a mono- or di-C1-7 alkylcarbamoyl, a C1-7 alkylsulfonylamino, phenylsulfonylamino, a C1-7 alkylsulfinyl, a C6-14 arylsulfanyl, a C6-14 arylsulfonyl, a C6-14 aryl, a 5-13-membered heteroaryl, a 5-10-membered heterocycloalkyl, a 5-10-membered heterocycloalkyl-C1-7 alkyl, or a 5-10-membered heterocycloalkyl-C1-7 alkoxy;
   ring C is a C3-10 cycloalkyl, a C5-10 cycloalkenyl, a C6-14 aryl, a 5-13-membered heteroaryl, or a 5-10-membered heterocycloalkyl;
   the alkyl, the alkenyl, the alkynyl, and the alkoxy are each independently unsubstituted or have one or more substituents selected from the group consisting of a halogen, hydroxy, cyano, nitro, amino, mercapto, a C1-7 alkyl, and a C2-7 alkynyl;
   the cycloalkyl, the cycloalkenyl, the aryl, the heteroaryl, and the heterocycloalkyl are each independently unsubstituted or have one or more substituents selected from the group consisting of a halogen, hydroxy, cyano, nitro, amino, mercapto, a C1-4 alkyl, and a C1-4 alkoxy; and
   the heteroaryl and the heterocycloalkyl each independently contain one or more heteroatoms selected from the group consisting of N, S, and O.

The process of obtaining the compound of Chemical Formula 2 from the compound of Chemical Formula 1 (step 1) may include: lithiating the compound of Chemical Formula 1 in a solvent and then carboxylating the compound of Chemical Formula 1 using carbon dioxide gas to obtain the compound of Chemical Formula 2.

The lithiation may be performed using a known organolithium reagent. In this case, the organolithium reagent is an organometallic compound containing a carbon-lithium bond. According to one exemplary embodiment of the present invention, organolithium reagents that may be used for lithiation include methyllithium, n-butyllithium, sec-butyllithium, isopropyllithium, tert-butyllithium, phenyllithium, and the like. For example, the organolithium reagent may be n-butyllithium.

The lithiation may be performed using 1 to 2 equivalents, for example, 1.5 to 2 equivalents of an organolithium reagent, relative to one equivalent of the compound of Chemical Formula 1. For example, 1.6 equivalents or more of *n*-butyllithium as the organolithium reagent may be used. The lithiation of the compound of Formula 1 may enhance reactivity by using tetramethylethylenediamine (TMEDA) in addition to the organolithium reagent, but the present invention is not limited thereto. The solvent used in the process of obtaining the compound of Chemical Formula 2 from the compound of Chemical Formula 1 (step 1) includes one or more selected from the group consisting of acetone, acetonitrile, dichloromethane, dimethylformamide, dimethylpropylene urea, dimethyl sulfoxide, ethyl acetate, hexamethylphosphoramide, tetrahydrofuran, pentane, hexane, heptane, ether, and diethyl ether.

After the lithiation of the compound of Chemical Formula 1, a carboxyl group is introduced using carbon dioxide. In this case, the yield may be maximized using CO₂ gas to prevent the inflow of moisture. Carboxylation using carbon dioxide gas may be performed at -60°C to 0°C, for example -60°C to -15°C. Preferably, the carboxylation may be performed at -60°C to -15°C to suppress heat generation, thereby maximizing the yield.

The process of obtaining the compound Chemical Formula 3 from the compound Chemical Formula 2 (step 2) may include:
converting a carboxyl group in the compound of Chemical Formula 2 to an acylazide group to obtain a compound of the following Chemical Formula 2a,
obtaining a compound of Chemical Formula 2b from the compound of Chemical Formula 2a, and
obtaining the compound of Chemical Formula 3 from the compound of Chemical Formula 2b: 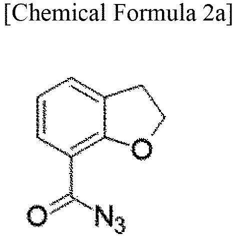 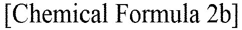 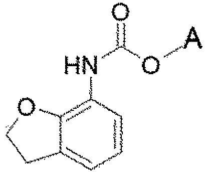
wherein A represents a C1-4 alkyl or a C1-4 alkoxy.

Step 2 may include: converting a carboxyl group in the compound of Chemical Formula 2 to an acylazide group to obtain a compound of the following Chemical Formula 2a, obtaining a compound of Chemical Formula 2b from the compound of Chemical Formula 2a (step 2-1), and obtaining the compound of Chemical Formula 3 from the compound of Chemical Formula 2b (step 2-2).

The solvent used in the process of synthesizing the compound of Chemical Formula 2b from the compound of Chemical Formula 2a and the process of obtaining the compound of Chemical Formula 3 from the compound of Chemical Formula 2b (step 2-1) may be selected from the group consisting of ethanol, propanol, butanol, and methoxyethanol. All of the solvents described above are applicable, but the use of *n*-propanol may be advantageous in consideration of the reaction temperature for producing the compound of Chemical Formula 2b and the ease of subsequent concentration and removal of the solvent. The solvent may be used in an amount that is 4- to 9-fold (w/v) the amount of the starting material.

The process of obtaining the compound of Chemical Formula 2b from the compound of Chemical Formula 2a and the process of obtaining the compound of Chemical Formula 3 from the compound of Chemical Formula 2b may be performed at 70°C to 115°C, but the present invention is not limited thereto. The preferred reaction temperature may be set to the reflux temperature of the solvent, depending on the choice of the solvent. According to one embodiment of the present invention, the process of obtaining the compound of Chemical Formula 2b from the compound of Chemical Formula 2a and the process of obtaining the compound of Chemical Formula 3 from the compound of Chemical Formula 2b are carried out in n-propanol. In this case, these processes may be carried out at a reaction temperature of 90°C to 100°C.

The process of synthesizing a compound of Chemical Formula 2b using n-propanol and then synthesizing a compound of Chemical Formula 3 from the compound of Chemical Formula 2b is illustratively expressed as the scheme below. 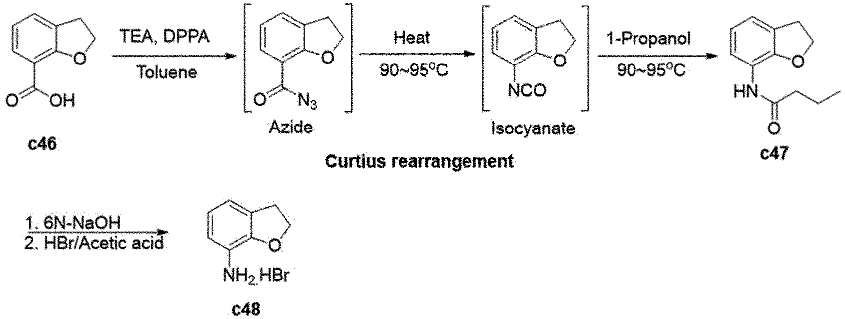
TEA: triethylamine
DPPA: diphenylphosphoryl azide

The azide compound of Chemical Formula 2a is produced from the compound of Chemical Formula 2 using TEA and DPPA through the Curtius rearrangement. When *n-*propanol is added *in situ* to the azide compound of Chemical Formula 2a and heated, the compound of Chemical Formula 2b is produced via an isocyanate compound (Chemical Formula 2a'). In this case, the azide and isocyanate intermediates are unstable and thus proceed *in situ.*

The process of obtaining the compound of Chemical Formula 3 from the compound of Chemical Formula 2b (step 2-2) includes: hydrolyzing the compound of Chemical Formula 2b with NaOH to obtain the compound of Chemical Formula 3. In this case, preferably, in order to effectively remove impurities that are also generated, a highly pure compound of Chemical Formula 3 may be obtained by forming an HBr salt at pH 1 to 2 using hydrobromic acid/acetic acid.

The process of obtaining the compound of Chemical Formula 4 from the compound of Chemical Formula 3 (step 3) may include: reacting the compound of Chemical Formula 3 in a solvent with N-halosuccinimide to obtain the compound of Chemical Formula 4.

Step 3 is a reaction step to selectively introduce two halogens at ortho and para positions. In this case, the equivalents of N-halosuccinimide, which is a source of halogen, the reaction temperature, and the reaction solvent are important factors. According to an embodiment of the present invention, bromine was used as the halogen. In this case, after monobromine is formed on the compound of Chemical Formula 3, it is important to minimize the production of tribromine while maximizing the production of the compound of Chemical Formula 4 corresponding to dibromine.

Solvents in which the compound of Chemical Formula 3 is dissolved thoroughly so that the reaction can be allowed to proceed in a homogeneous state are preferred as the reaction solvent. Such a reaction solvent may include one or more solvent selected from the group consisting of ethanol, N,N-dimethylformamide, N,N-dimethyl acetamide (DMAc), and N-methylpyrrolidone (NMP), but the present invention is not limited thereto. The solvent may be used in an amount that is 10- to 40-fold (w/v) the amount of the compound of Chemical Formula 3.

According to one exemplary embodiment of the present invention, the solvent is ethanol and may be used in an amount that is 30- to 40-fold (w/v) the amount of the compound of Chemical Formula 3, but the present invention is not limited thereto.

N-halosuccinimide may be used in an amount of 2 to 2.3 equivalents relative to the compound of Chemical Formula 3, and may be preferably used in an amount of 2.05 to 2.15 equivalents to minimize the generation of impurities.

In the process of obtaining the compound of Chemical Formula 4 from the compound of Chemical Formula 3 (step 3), the reaction temperature may range from -10°C to 30°C, and the reaction may be preferably performed at -10°C to 5°C to minimize the generation of impurities. Meanwhile, the process of obtaining the compound of Chemical Formula 5 from the compound of Chemical Formula 4 (step 4) may include: halogenating the amine of the compound of Chemical Formula 4 through the Sandmeyer reaction. The Sandmeyer reaction may include: reacting the compound of Chemical Formula 4 with NaNO₂ to form a diazonium salt of the compound of Chemical Formula 4, and reacting the diazonium salt with a transition metal salt to obtain the compound of Chemical Formula 5, but is not limited thereto. The transition metal salt refers to a salt of a transition metal that includes, but is not limited to, copper, iron, and cobalt. For example, the transition metal salt may be a copper salt including CuCl and CuBr.

The NaNO₂ may be used in an amount of 1 to 2 equivalents relative to the compound of Chemical Formula 4, but the present invention is not limited thereto.

Meanwhile, the reaction between the diazonium salt of the compound of Chemical Formula 4 and the transition metal salt may be performed at 30°C to 80°C, for example, 40°C to 70°C, or 50°C to 60°C. In order to minimize non-halogenated impurities, the reaction may be preferably performed at 50°C to 60°C.

To purify impurities, the reaction product may be washed with a solvent. In this case, ethanol, 1-propanol, isopropyl alcohol, and the like may be used as the solvent. Step 4 may further include purification, preferably with isopropyl alcohol.

The process of obtaining the compound of Chemical Formula 6 from the compound of Chemical Formula 5 (step 5) may include: selectively converting the substituent X in the compound of Chemical Formula 5 to an aldehyde group using an organomagnesium reagent and dimethylformamide (DMF) as reaction reagents. Here, the organomagnesium reagent is a compound expressed as RMgX (where R is an alkyl or an allyl, and X is a halogen).

According to one embodiment of the present invention, as one example of the organomagnesium reagent, isopropylmagnesium chloride converts X at the ortho position of the compound of Chemical Formula 5 to MgX to form a Grignard reagent, which is converted to an aldehyde group by reaction with DMF.

The organomagnesium reagent may be used in an amount of 1 to 2 equivalents, preferably 1 to 1.7 equivalents, relative to one equivalent of the compound of Chemical Formula 5, but the present invention is not limited thereto. DMF may be used in an amount of 3 to 6 equivalents, preferably 4 to 5 equivalents, relative to one equivalent of the compound of Chemical Formula 5.

In step 5, tetrahydrofuran (THF), diethyl ether, 2-methyl tetrahydrofuran, and the like may be used as the reaction solvent. The reaction solvent may be used in an amount that is 5- to 15-fold (w/v), preferably 8- to 12-fold (w/v), the amount of the compound of Chemical Formula 5, but the present invention is not limited thereto.

The reaction of step 5 may be performed at -10°C to 50°C, for example, 0°C to 50°C, 10°C to 50°C, 20°C to 50°C, or 30°C to 50°C. In order to minimize the production of related materials and reaction intermediates, the reaction may be preferably performed at 30°C to 50°C. However, because there is an increase in temperature due to heat generation when DMF is added, the starting reaction temperature preferably ranges from 25°C to 30°C, which is lower than the above reaction temperature.

The process of reacting of the compound of Chemical Formula 7 with the compound of Chemical Formula 6 to obtain the compound of Chemical Formula 8 (step 6) may include: lithiating a halogen position of the compound of Chemical Formula 7 and coupling the same with the compound of Chemical Formula 6. The lithiation may be performed using an organolithium reagent as described above. The lithiation may be performed using 1 to 1.3 equivalents of the organolithium reagent relative to one equivalent of the compound of Chemical Formula 7, but the present invention is not limited thereto. For example, *n*-BuLi may be used. When the amount of organolithium reagent is excessive compared to the compound of Chemical Formula 7, the organolithium reagent may react with the halogen present in the compound of Chemical Formula 6. Therefore, the organolithium reagent may be used in an appropriate equivalent amount. Also, in order to maintain the stability of the lithium salt to be produced, it is preferable to add butylmagnesium chloride or *tert-*butylmagnesium chloride to the compound of Chemical Formula 7 in a solvent prior to the lithiation reaction. It was confirmed that when only the organolithium reagent is used, two types of impurities remaining in the finally produced compound of Chemical Formula 9 are produced in an amount from 1% to 9% depending on the equivalents of the organolithium reagent used, whereas when butylmagnesium chloride or *tert*-butylmagnesium chloride, which may form a complex with the lithium salt to further stabilize the lithium salt, is used, the impurities could be reduced to 0 to 1% depending on the equivalents of butylmagnesium chloride or *tert*-butylmagnesium chloride used. Butylmagnesium chloride or *tert-*butylmagnesium chloride may be used in an amount of 0.2 to 1 equivalent, for example, 0.5 to 1 equivalent, or 0.5 to 0.7 equivalents, relative to one equivalent of the compound of Chemical Formula 7.

The reacting of the compound of Chemical Formula 7 with the compound of Chemical Formula 6 to obtain the compound of Chemical Formula 8 may include: obtaining a compound of Chemical Formula 7' from the compound of Chemical Formula 7 and coupling the compound of Chemical Formula 7' to the compound of Chemical Formula 6.

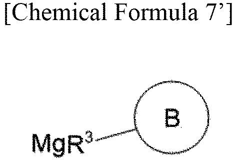

The process of obtaining the compound of Chemical Formula 9 from the compound of Chemical Formula 8 (step 7) may be performed using known methods. The compound of Chemical Formula 9 may be obtained by reducing the compound of Chemical Formula 8 according to the information disclosed in Korean Patent Publication No. 2017-0142904 (Patent Document 2), but the present invention is not limited thereto.

According to the above method, the compound of Chemical Formula 9, which corresponds to an important intermediate for the compound of Chemical Formula I as an SGLT inhibitor, and the compound of Chemical Formula I, which is a final target compound, may be obtained with high yield and high quality through a total of 7 short reaction steps using the compound of Chemical Formula 1, which is more easily commercially available, as a starting material. Also, the method for producing an SGLT inhibitor has excellent economic efficiency because the SGLT inhibitor may be produced without special equipment such as an ozone generator.

According to another exemplary embodiment of the present invention, the compound of Chemical Formula 9 may be produced using a method which includes: obtaining a compound of Chemical Formula 11 from a compound of Chemical Formula 10; reacting a compound of Chemical Formula 12 with the compound of Chemical Formula 11 to obtain a compound of Chemical Formula 13; and obtaining the compound of Chemical Formula 9 from the compound of Chemical Formula 13.

The present invention provides a method for producing a compound of Chemical Formula 9, which includes the following steps:
obtaining a compound of Chemical Formula 11 from a compound of Chemical Formula 10;
reacting a compound of Chemical Formula 12 with the compound of Chemical Formula 11 to obtain a compound of Chemical Formula 13; and
obtaining the compound of Chemical Formula 9 from the compound of Chemical Formula 13: 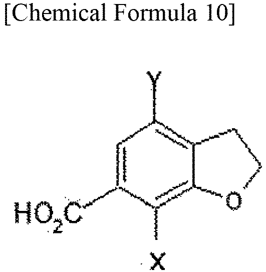 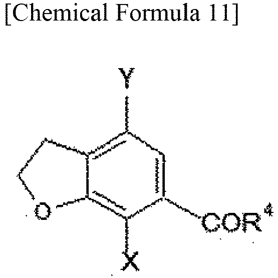 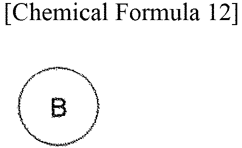 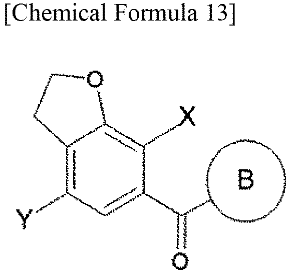 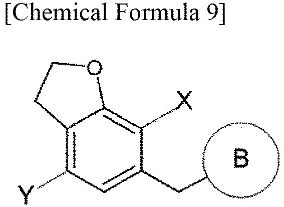
wherein:
   X, Y, and R⁴ are each independently a halogen, and
   B is 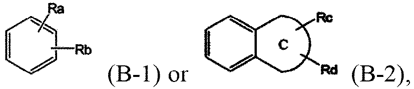
   wherein Ra, Rb, Rc, and Rd are each independently hydrogen, a halogen, hydroxy, mercapto, cyano, nitro, amino, carboxy, oxo, a C1-7 alkyl, a C1-7 alkylthio, a C2-7 alkenyl, a C2-7 alkynyl, a C1-7 alkoxy, a C1-7 alkoxy-C1-7 alkyl, a C2-7 alkenyl-C1-7 alkyloxy, a C2-7 alkynyl-C1-7 alkyloxy, a C3-10 cycloalkyl, a C3-7 cycloalkylthio, a C5-10 cycloalkenyl, a C3-10 cycloalkyloxy, a C3-10 cycloalkyloxy-C1-7 alkoxy, a phenyl-C1-7 alkyl, a C1-7 alkylthio-phenyl, a phenyl-C1-7 alkoxy, a mono- or di-C1-7 alkylamino, a mono- or di-C1-7 alkylamino-C1-7 alkyl, a C1-7 alkanoyl, a C1-7 alkanoylamino, a C1-7 alkylcarbonyl, a C1-7 alkoxycarbonyl, carbamoyl, a mono- or di-C1-7 alkylcarbamoyl, a C1-7 alkylsulfonylamino, phenylsulfonylamino, a C1-7 alkylsulfinyl, a C6-14 arylsulfanyl, a C6-14 arylsulfonyl, a C6-14 aryl, a 5-13-membered heteroaryl, a 5-10-membered heterocycloalkyl, a 5-10-membered heterocycloalkyl-C1-7 alkyl, or a 5-10-membered heterocycloalkyl-C1-7 alkoxy;
   ring C is a C3-10 cycloalkyl, a C5-10 cycloalkenyl, a C6-14 aryl, a 5-13-membered heteroaryl, or a 5-10-membered heterocycloalkyl;
   the alkyl, the alkenyl, the alkynyl, and the alkoxy are each independently unsubstituted or have one or more substituents selected from the group consisting of a halogen, hydroxy, cyano, nitro, amino, mercapto, a C1-7 alkyl, and a C2-7 alkynyl;
   the cycloalkyl, the cycloalkenyl, the aryl, the heteroaryl, and the heterocycloalkyl are each independently unsubstituted or have one or more substituents selected from the group consisting of a halogen, hydroxy, cyano, nitro, amino, mercapto, a C1-4 alkyl, and a C1-4 alkoxy; and
   the heteroaryl and the heterocycloalkyl each independently contain one or more heteroatoms selected from the group consisting of N, S, and O.

The process of obtaining the compound of Chemical Formula 11 from the compound of Chemical Formula 10 is a process of halogenating a carboxyl group in the compound of Chemical Formula 10. The compound of Chemical Formula 10 is dissolved in a solvent, and reacted with a halogenation reagent in the presence or absence of a catalyst. Dichloromethane, dichloroethane, and the like may be used as the solvent. Oxalyl halide, thionyl halide, and the like may be used as the halogenation reagent, but the present invention is not limited thereto. When oxalyl halide is used as the halogenation reagent, dimethylformamide may be used as the catalyst, but the present invention is not limited thereto. In this case, the halogenation reagent may be used in an amount of 0.1% by weight to 1% by weight based on the compound of Chemical Formula 10.

To minimize side reactions, the process of reacting of the compound of Chemical Formula 11 with the compound of Chemical Formula 12 to obtain the compound of Chemical Formula 13 may be performed at a reaction temperature of -35°C to -10°C. Preferably, the reaction may be performed at a reaction temperature of -20°C to -10°C. The compound of Chemical Formula 12 may be used in an amount of 1 to 3 equivalents, preferably 2 to 3 equivalents, and more preferably 2 to 2.6 equivalents, relative to the compound of Chemical Formula 11, but the present invention is not limited thereto. For the reaction between the compound of Chemical Formula 11 and the compound of Chemical Formula 12, a Lewis acid such as AlCl₃, FeCl₃, BiCl₃, ZnClz, Fe₂O₃, and the like may be used. Preferably, AlCl₃ may be used. The Lewis acid may be used in an amount of 0.9 to 1.5 equivalents, preferably 1.0 to 1.2 equivalents, relative to the compound of Chemical Formula 11, but the present invention is not limited thereto.

The compound of Chemical Formula 9 may be produced by reducing the compound of Chemical Formula 13.

Because the compound of Chemical Formula 12 is cheaper and has easier quality control compared to the compound of Chemical Formula 7, the use of the compound of Chemical Formula 12 may be advantageous compared to that of the compound of Chemical Formula 7.

Meanwhile, the compound of Chemical Formula 10 may be produced from 1) the compound of Chemical Formula 5, 2) the compound of Chemical Formula 6, or 3) a compound of Chemical Formula 14.

According to one exemplary embodiment, the compound of Chemical Formula 10 may be obtained by carboxylating the compound of Chemical Formula 5: 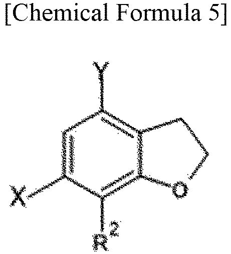 wherein:
R², X, and Y are each independently a halogen.
According to another exemplary embodiment, the compound of Chemical Formula 10 may be obtained by converting an aldehyde group in the compound of Chemical Formula 6 to a carboxyl group: 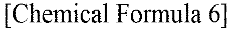 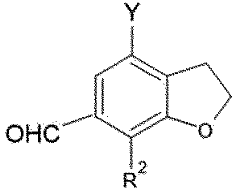
wherein:
   R² and Y are each independently a halogen.

According to still another exemplary embodiment, the compound of Chemical Formula 10 may be obtained by carboxylating the compound of Chemical Formula 14: 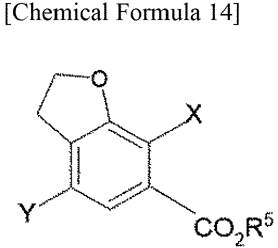 wherein:
X and Y are each independently a halogen, and
R⁵ is a C1-4 alkyl.

Meanwhile, the compound of Chemical Formula 6 may be synthesized from the compound of Chemical Formula 5 or the compound of Chemical Formula 14.

According to one exemplary embodiment of the present invention, the compound of Chemical Formula 6 may be obtained by selectively converting the substituent X in the compound of Chemical Formula 5 to an aldehyde group. This process is as described above: 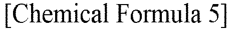 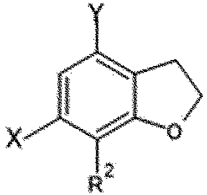 wherein:
R², X, and Y are each independently a halogen.

According to yet another exemplary embodiment, the compound of Chemical Formula 6 may be obtained by:
obtaining a compound of Chemical Formula 15 from the compound of Chemical Formula 14, and
obtaining the compound of Chemical Formula 6 from the compound of Chemical Formula 15: 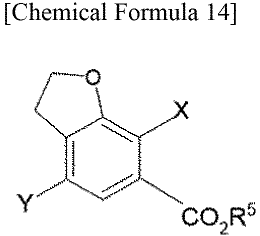 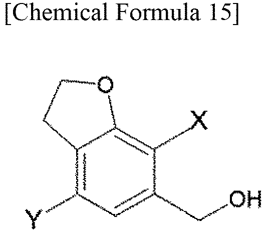
wherein:
   X and Y are each independently a halogen, and
   R⁵ is a C1-4 alkyl.

In the methods of producing a compound of Chemical Formula 10 from 1) a compound of Chemical Formula 5, 2) a compound of Chemical Formula 6, or 3) a compound of Chemical Formula 14 and producing a compound of Chemical Formula 9 from the compound of Chemical Formula 10, the compound of Chemical Formula 12 is used. The compound of Chemical Formula 12 is cheaper than the compound of Chemical Formula 7 used in the conventional schemes, and the use of the compound of Chemical Formula 12 may address problems caused by the impurities generated during the production of Chemical Formula 7, thereby obtaining the high-quality compound of Chemical Formula 9 and the final target material.

### [Advantageous Effects]

According to the present invention, the compound of Chemical Formula 9, which corresponds to an important intermediate for the compound of Chemical Formula I as an SGLT inhibitor, can be obtained with high yield and high quality. In the method for producing an SGLT inhibitor according to the present invention, the SGLT inhibitor can be produced without special equipment such as an ozone generator, thereby significantly improving economic efficiency.

### [Best Mode]

Hereinafter, the present invention will be described in more detail with reference to examples thereof. However, it should be understood that the following examples are only for the purpose of describing the present invention and are not intended to illustrate the scope of the present invention.

### [Abbreviations]

The meanings of the abbreviations described in the examples below are as follows.
- Ac₂O: acetic anhydride
- BF₃OEt₂: boron trifluoride etherate
- n-BuLi: normal butyllithium
- DIPEA: N,N-diisopropylethylamine
- DMAP: 4-dimethylaminopyridine
- DMF: N,N-dimethylformamide
- DCM: dichloromethane
- DPPA: diphenylphosphoryl azide
- EA: ethyl acetate
- EtOH: ethanol
- Hex: hexane
- MeOH: methanol
- NBS: N-bromosuccinamide
- OX: oxalyl chloride
- THF: tetrahydrofuran
- HCl: hydrochloride
- HBr: hydrobromide
- TMEDA: tetramethylethylenediamine

Examples 1 and 2 below illustrate a process of synthesizing compound c40, which is a preferred example of the compound of Chemical Formula 9, and Example 3 illustrates a process of synthesizing compound c28, which is a preferred example of the compound of Chemical Formula I, from compound c40. Also, Example 4 illustrates a process of synthesizing c40 centered on c51.

Scheme 3 below schematically shows a process of synthesizing compound c40 exemplified in Examples 1 and 2.

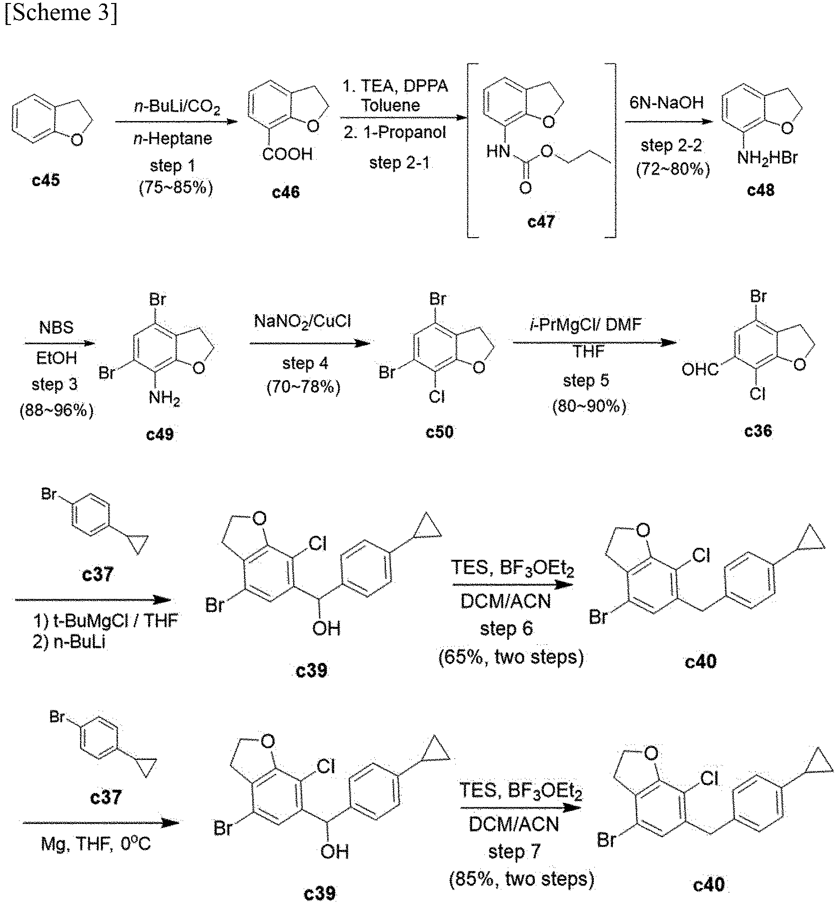

### [EXAMPLES]

### Example 1: Synthesis of 4-bromo-7-chloro-6-(4-cyclopropylbenzyl)-2,3-dihydrobenzofuran (Compound 40)

### Step 1: 2,3-Dihydrobenzofuran-7-carboxylic acid (Compound 46)

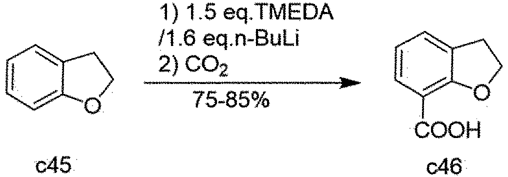

n-Heptane (12 L) was added to a reactor, and 2,3-dihydrobenzofuran (c45: 1.2 kg, 1.0 eq) and TMEDA (1.74 kg, 1.5 eq) were added thereto and cooled to 5°C to 15°C. *n-*BuLi (6.4 L, 1.6 eq) was added dropwise to the cooled mixture while maintaining the temperature between 5°C to 15°C. The reaction mixture was stirred for 30 minutes and then cooled to -40°C to -55°C. CO₂ (gas) was input into the cooled reaction mixture. At this time, the temperature rose to -10°C, and the gas was added until the temperature no longer rose. After it was confirmed through HPLC that the reaction was completed, water (136 L) was added to terminate the reaction, and MTBE (12 L) was added, stirred for 30 minutes, and left to stand. The separated aqueous layer was transferred to another reactor, and c-HCl was added dropwise to adjust the pH to 1 to 2 to precipitate crystals. The temperature of the crystallization solution was adjusted to 5°C to 15°C, and stirred for 2 hours. The temperature of the crystallization solution was again lowered to 0°C to 5°C, filtered, and then washed with water (4.8 L). The filtrate was dried at 60°C to 70°C for 48 hours to obtain the yellow title compound (1.29 kg, 79%).

¹H NMR (500 MHz, CDCl₃) δ9.52(s, 1H), 7.84(d, *J* = 7.9 Hz, 1H), 7.38(t, 1H), 6.95(m, 1H), 4.76(m, 2H), 3.29(t, *J* = 8.5 Hz, 2H); [M+H]⁺ 165

### Step 2: 2,3-Dihydrobenzofuran-7-amine hydrobromide (Compound 48)

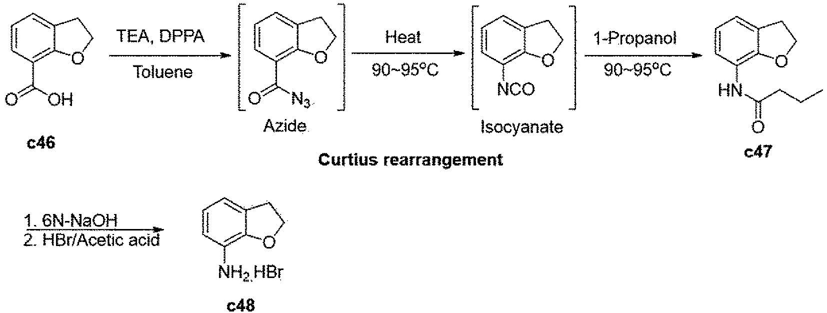

*n*-Propanol, 2-methoxyethanol, *n*-butanol, and ethanol were applicable as the solvents used for the synthesis of compound c47 and hydrolysis to amines.

**[Table 1]**

| Types of alcohol | Reaction temperature | Reaction solution purity |
|---|---|---|
| Ethanol | 70 to 75°C | 83.6% |
| *n*-Propanol | 95 to 97°C | 88.7% |
| 2-Methoxyethanol | 110 to 115°C | 88.8% |
| *n*-Butanol | 110 to 115°C | 90.0% |

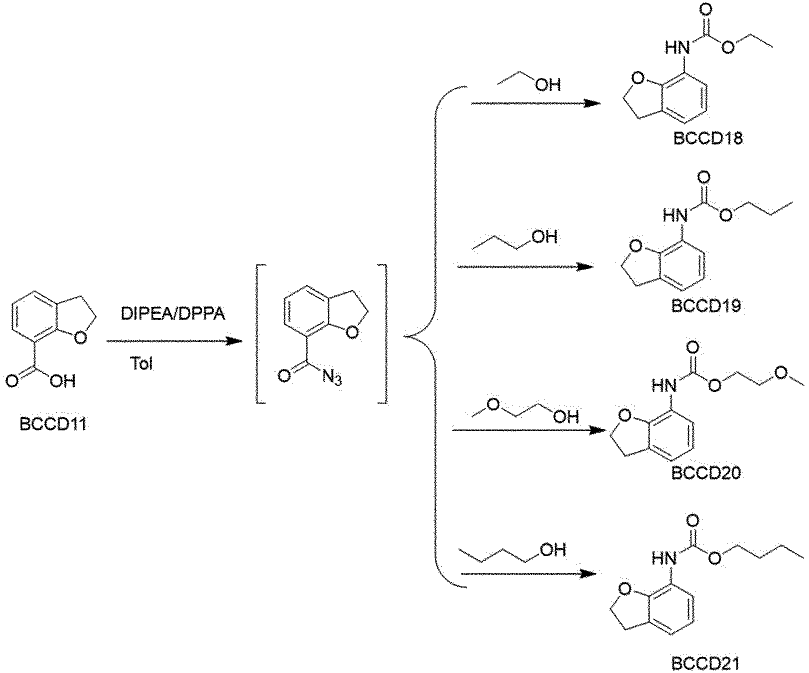

However, *n*-propanol was selected as the solvent in consideration of the reaction temperature used to produce the compound of Chemical Formula 2b and the ease of subsequent concentration and removal of the solvent.

*n*-Propanol, which was used as a reactant and solvent for isocyanate, was used in an amount that was 4- to 8-fold, preferably 6-fold, the amount of compound c2 in order to minimize the formation of urea impurities, and although it is possible for the reaction temperature to range from 70°C to 115°C, preferably, the reaction temperature ranges from 90°C to 97°C, which is the reflux temperature of *n*-propanol.

A preferred synthesis example according to the above results is as follows.

Toluene (12.5 L), 2,3-dihydrobenzofuran-7-carboxylic acid (c46: 2.5 kg, 1.0 eq), and triethylamine (1.7 kg, 1.1 eq) were added to a reactor and cooled to 0°C to 5°C. DPPA (4.9 kg, 1.2 eq) was added to the mixture, stirred for 30 minutes, and then heated to 25°C to 35°C while stirring. The reaction was continued until 2% or less of C46 remained. 1-Propanol (15 L) was added to another reactor and heated to 95°C to 100°C. The above reaction solution was added to the heated 1-propanol solution and stirred at 90°C to 95°C for 30 minutes. After it was confirmed that the content of the reaction intermediate was 1% or less, the reaction solvent was concentrated and removed. 6 N NaOH (325 L) was added to the concentrated reaction solution and stirred at 90°C to 100°C for 3 to 5 hours. When 1% or less of c47 remained, the organic solvent was removed by concentration under reduced pressure, cooled to 20°C to 30°C, and extracted four times with toluene (5 L). The extracted toluene layers were combined and completely concentrated. The concentrated toluene layer was cooled to 10°C to 20°C and then dissolved by adding toluene (17 L). A hydrobromic acid/acetic acid solution was added to the dissolution solution to proceed with crystallization while adjusting the pH to 1 to 2. Then, the mixed solution was cooled to 10°C to 20°C and stirred for an hour. The resulting crystals were filtered and then washed with toluene (3.2 L). The obtained crystals were dried at 60°C to 70°C for 18 hours to obtain the yellow title compound (2.5 kg, 77%).

¹H NMR (500 MHz, DMSO d6+D₂O) δ 7.48 - 6.56 (m, 3H), 4..62 (m, J = 9.2 Hz, 2H), 3.21 (t, J = 8.5 Hz, 2H); [M+H]⁺ 136

### Step 3: 4,6-Dibromo-2,3-dihydrobenzofuran-7-amine (c49)

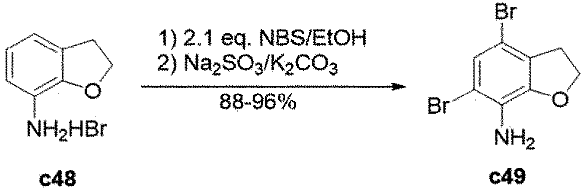

Step 3 is a reaction to selectively introduce two bromines at the ortho and para positions. In this case, the equivalents of NBS, which is a source of bromine, the reaction temperature, and the reaction solvent were important factors. After the formation of monobromine, it was important to minimize the production of tribomine while maximizing the production of dibromine, which is the title compound. The reaction solvent was selected from solvents that could dissolve c48 thoroughly and proceed the reaction in a homogeneous state. EtOH, DMF, DMAc, and NMP may be used as the solvent. These solvents were able to minimize the production of tribromide, and EtOH was selected in consideration of the highest yield, the minimum tribromide production, and the purification of impurities, and was used in an amount that was 30-fold the amount of the solvent used to dissolve c48 thoroughly.

**[Table 2]**

| Solvent type (amount used) | Compound 49 | Ethoxylated impurities | Monobromide | Tribromide |
|---|---|---|---|---|
| EtOH (30-fold) | 82.9% | 5.1% | 5.9% | 0.2% |
| DMF (10-fold) | 77.9% | NA | 8.3% | NA |
| DMAc (10-fold) | 67.9% | NA | 10.9% | 0.5% |
| NMP (10-fold) | 61.5% | NA | 17.2% | 0.2% |

| | | | | |
|---|---|---|---|---|
| *, The ethoxylated impurities and monobromide impurities in Table 2 were removed during the subsequent purification process | | | | |

A preferred synthesis example based on the above results is as follows.

Ethanol (36 L) and 2,3-dihydrobenzofuran-7-amine hydrobromide (c48: 1.2 kg, 1.0 eq) were added to a reactor and dissolved at 20°C to 30°^{C} until completely dissolved. The mixture was cooled to -10°C, and NBS (2.1 kg, 2.1 eq) was then added in portions. The reaction temperature was raised to 0°C to 5°C, and stirring was performed for 30 minutes. When it was confirmed that less than 5% of monobromine remained, the reaction solution was added to an aqueous solution obtained by adding K₂CO₃ (1.2 kg, 1.0 wt/wt) and Na₂S₂O₃ (1.2 kg, 1.0 wt/wt) to water (12 L), and the reaction was then terminated. The reaction solution was washed with ethanol (1 L) and put into the aqueous solution. After the reaction solution was stirred for 30 minutes, ethanol was removed by vacuum concentration. After the temperature was lowered to 15°C to 25°C, the crystals were then filtered. The crystals were washed with water (6 L), and then dried under vacuum at 50°C to 60°C for 16 hours to obtain the title compound (1.5 kg, 92%).

¹H NMR (61 MHz, CDCl₃) δ7.07(s, 1H), 4.65(t, *J =* 8.5 Hz, 2H), 3.91(s, 2H), 3.17(t, *J =* 8.5 Hz, 2H); [M+H]⁺ 294

### Step 4: 4,6-Dibromo-7-chloro-2,3-dihydrobenzofuran (c50)

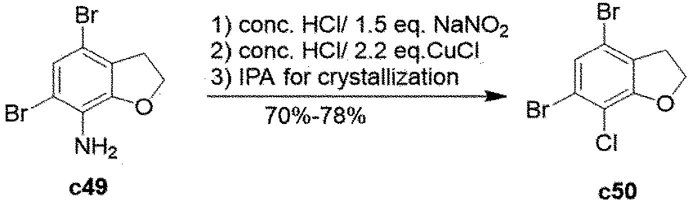

HCl (15 L) was added to a reactor, and the temperature was lowered to 0°C to 5°C. Thereafter, 4,6-dibromo-7-chloro-2,3-dihydrobenzofuran (c49: 3.0 kg, 1 eq) was added thereto and stirred for 10 minutes. Then, a solution obtained by dissolving sodium nitrite (1.1 kg, 1.5 eq) in water (2 L) was added dropwise to the reactor and stirred at 0°C to 5°C for 30 minutes. As the crystals dissolved, the red solution turned black. c-HCl (12.6 L) was added to another reactor, and copper (I) chloride (2.2 kg, 2.2 eq.) was added thereto. Then, the mixture was heated to 50°C to 60°C, and the reaction solution was added thereto and stirred at 50°C to 60°C for 30 minutes to terminate the reaction. After it was confirmed that the reaction was completed, the reaction mixture was cooled to 0°C to 10°C, and water (20 L) was added to terminate the reaction. The mixture was stirred for 30 minutes, filtered, and washed with water (5 kg). Then, the filtered crystals were dissolved in DCM (20 L). To remove the color, silica gel (2 kg) was placed on a filter, and the DCM solution was added, filtered, and washed with DCM (5 L). The filtrate was concentrated under reduced pressure to remove DCM, and IPA (6 L) was added and re-concentrated to completely remove the remaining DCM. IPA (12 L) was added to the concentrate, and then completely dissolved by heating to 70 to 80°C. The dissolution solution was cooled again to 0°C to 10°C, stirred for 2 hours, and then filtered at the same temperature. The filtrate was washed with IPA (3 L) cooled to 5°C to 10°C and dried under vacuum at 50°C to 60°C for 16 hours to obtain the title compound as purple-red crystals (2.27 kg, 71 %).

¹H NMR (500 MHz, DMSO) δ7.32(s, 1H), 4.72(t, *J* = 7.3 Hz, 1H), 3.22(t, *J* = 9.2 Hz, 1H); [M+H]⁺ 313

### Step 5: 4-Bromo-7-chloro-2,3-dihydrobenzofuran-6-carbaldehyde (c36)

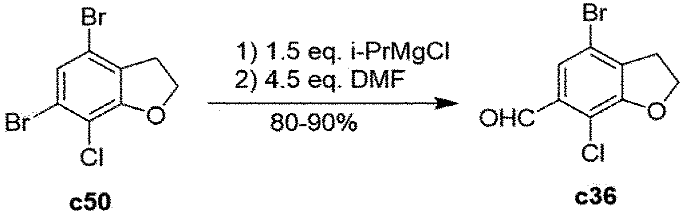

Step 5 is a reaction step to selectively convert bromine at the ortho position to aldehyde, and the amounts of *i*-PrMgCl and DMF used and the reaction temperature were important factors. The amount of *i*-PrMgCl used may be 1.0 to 1.7 equivalents, preferably 1.5 to 1.6 equivalents, and the amount of DMF used may be 3.5 to 5.5 equivalents, preferably 4.5 to 5.5 equivalents. Although it was possible for the reaction temperature to range from - 10°C to 50°C, 30°C to 50°C was preferred. However, because the temperature increased due to heat generation when DMF was added, 25°C to 30°C was selected as the starting reaction temperature.

**[Table 3]**

| Number | Reaction temperature | C36 | Related material | Reaction intermediate |
|---|---|---|---|---|
| 1 | -10°C | 74.8% | 3.6% | 15.9% |
| 2 | 5 to 10°C | 74.5% | 10.0% | 10.4% |
| 3 | 30°C | 84% | 2.6% | 9.2% |
| 4 | 50°C | 84% | 0.2% | 7.6% |

A preferred synthesis example based on the above results is as follows. THF (22.5 L) was added to a reactor, and 4,6-dibromo-7-chloro-2,3-dihydrobenzofuran (c50: 2.25 kg, 1.0 eq) was added thereto and dissolved. After the air was replaced with nitrogen three times, the solution was cooled to 0°C to 5°C. Isopropylmagnesium chloride (5.1 kg, 5.6 L, 2 M in THF, 1.5 eq.) was added dropwise to the solution and then stirred for 30 minutes. The temperature of the reaction solution was raised again to 25 to 30°C, DMF (2.4 kg, 4.5 eq) was added, and stirred at 25 to 30°C for 30 minutes. After the completion of the reaction was confirmed, the reaction solution was cooled to 0°C to 10°C. Acetic acid (2.1 L) was added to adjust the pH to 4 to 6, and water (22.5 L) was then added to terminate the reaction. After the reaction was completed, the solution was concentrated under vacuum to completely remove THF, cooled again to 0°C to 10°C, and then filtered. The filtrate was washed with water (6.7 L). The resulting crystals were added to the reactor along with *n*-heptane (17 L), heated to 90°C, and stirred for 30 minutes. The solution was cooled again to 0°C to 10°C, and stirred for 2 hours, and then the crystals were filtered and washed with *n*-heptane (4.5 L). The obtained crystals were dried under vacuum at 50°C to 60°C for 16 hours to obtain the yellow title compound (1.6 kg, 85%).

¹H NMR (500 MHz, CDCl₃) δ10.34(s, 1H), 7.60(s, 1H), 4.80(t, *J* = 9.2 Hz, 1H), 3.35(t, *J =* 9.2 Hz, 1H); [M+H]⁺ 263

### Step 6: 4-Bromo-7-chloro-2,3-dihydrobenzofuran-6-yl)(4-cyclopropylphenyl)methanol (c39)

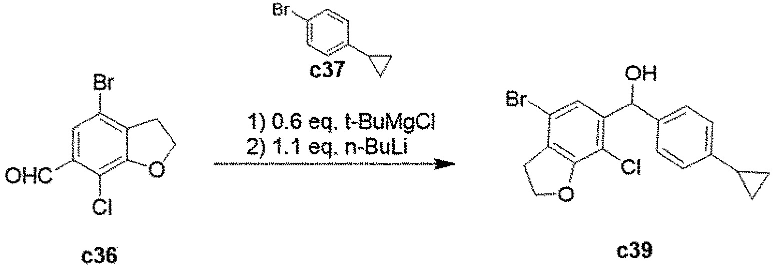

THF (800 mL) was added to a reactor, and 1-bromo-4-cyclopropylbenzene (c37: 98.0 g, 497.2 mmol) was added thereto and dissolved. After the air was replaced with nitrogen three times, the solution was cooled to -10°C. t-Butylmagnesium chloride (114.8 mL, 229.5 mmol, 2 M in THF, 0.6 eq.) was slowly added dropwise. The mixture was stirred at the same temperature for 10 minutes and then cooled to -60°C to -70°C. n-Butyl lithium (176 mL, 2.5M in Hex, 439.8 mmol1.15 eq.) was added dropwise to the cooled reaction solution, and 4-bromo-7-chloro-2,3-dihydrobenzofuran-6-carbaldehyde (Compound 36: 100 g, 382.4 mmol 1.0 eq.) dissolved in THF (1600 mL) was added dropwise and stirred for 30 minutes. After the completion of reaction was confirmed, an aqueous 1 N HCl (1,000 mL) solution was added to terminate the reaction. After the reaction was completed, the reaction solution was concentrated under reduced pressure to remove THF. After DCM (1,000 mL) was added to the concentrate and stirred for 10 minutes, the layers were separated. The separated DCM layer was concentrated under reduced pressure and used to produce the next compound, 4-bromo-7-chloro-6-(4-cyclopropylbenzyl)-2,3-dihydrobenzofuran (c40), without further purification.

¹H NMR (400 MHz, CDCl₃) δ7.33(s, 1H), 7.25(d, *J* = 7.6 Hz, 2H), 7.02(d, 8.1 Hz 1H), 6.07(s, 1H), 4.69(m, 2H), 3.27(m, 2H), 1.85(m, 1H), 0.93(m, 2H), 0.66(m, 2H); [M+H]⁺ 281

### Step 7: 4-Bromo-7-chloro-6-(4-cyclopropylbenzyl)-2,3-dihydrobenzofuran (c40)

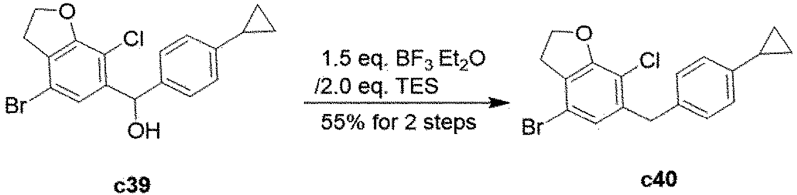

DCM (1,000 mL) was added and dissolved in concentrated 4-bromo-7-chloro-2,3-dihydrobenzofuran-6-yl)(4-cyclopropylphenyl)methanol (c39), and acetonitrile (1,000 mL) was added thereto. After the reaction solution was cooled to -20°C, BF₃OEt₂ (70.8 mL, 573.6 mmol, 1.5 eq) and triethylsilane (122 mL, 764.8 mmol, 2.0 eq) were added and stirred at -20°C to -25°C for an hour. After the temperature was raised to 20 to 30°C, the reaction solution was stirred for an hour. After the completion of the reaction was confirmed, a saturated NaHCO₃ solution (1,000 mL) was added to the reaction solution to terminate the reaction. After the reaction was completed, the reaction solution was concentrated under reduced pressure until it reached 1,000 mL, and then DCM (1,000 mL) was added and stirred for 10 minutes, and the layers were separated. Na₂SO₄ was added to the organic layer to remove moisture, and then the organic layer was filtered and concentrated. DCM (50 mL) was added to the concentrate and dissolved. Then, methanol (800 mL) was added, and crystallized at 20 to 30°C for 2 hours. The crystallization solution was filtered, washed with methanol (100 mL), and dried under vacuum at 40°C to 50°C to obtain the off-white title compound (90.0 g, 64.7%, steps 6 and 7 integrated).

¹H NMR (400 MHz, CDCl₃) δ7.07(d, J = 8.0 Hz, 2H), 6.99(d, J = 8.0 Hz, 2H), 6.80(s, 1H), 4.70(t, J = 8.8 Hz, 2H), 3.97(s, 2H), 3.26(t, J = 8.8 Hz, 2H), 1.88-1.84(m, 1H), 0.95-0.90(m, 2H), 0.68-0.64(m, 2H); [M+H]⁺ 365

### Example 2: Synthesis of 4-bromo-7-chloro-6-(4-cyclopropylbenzyl)-2,3-dihydrobenzofuran (Compound 40)

### Step 6: 4-Bromo-7-chloro-2,3-dihydrobenzofuran-6-yl)(4-cyclopropylphenyl)methanol (Compound 39)

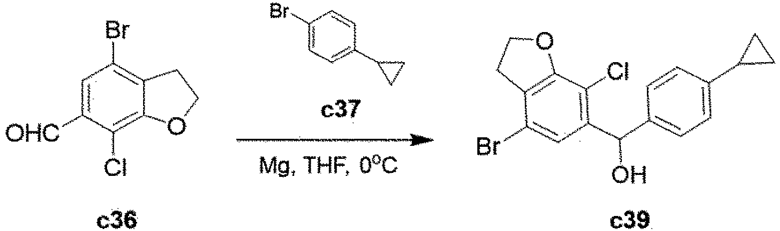

Production of (4-cyclopropylphenyl)magnesium bromide (Compound c38): A 250 mL 3-necked flask containing magnesium (fragment, 1.1 g, 46.6 mmol) was flame-dried. The flask was equipped with a condenser and an addition funnel under a nitrogen atmosphere. 4-Cyclopropylphenyl bromide (Compound c37) (6.0 mL, 42.4 mmol) in anhydrous THF (32.4 mL) was transferred to the addition funnel. The Grignard reaction was initiated using approximately 5 mL of a 4-cyclopropylphenyl bromide solution. The remaining bromide solution was added to the flask at room temperature for 4 hours. The resulting solution was used directly in the next step.

A freshly prepared solution of (4-cyclopropylphenyl)magnesium bromide (Compound c38) (0.85 M in THF, 30.0 mL, 26.4 mmol) was added to a solution of 4-bromo-7-chloro-2,3-dihydrobenzofuran-6-carbaldehyde (Compound c36) (4.6 g, 17.6 mmol) in anhydrous THF (170 mL) at 0°C under a nitrogen atmosphere. The reaction mixture was stirred at 0°C for 30 minutes. Water (100 mL) was added to the reaction mixture to terminate the reaction, and the reaction mixture was extracted with ethyl acetate (100 mL). The organic layer was dried over MgSO₄, filtered, and concentrated under vacuum to obtain the crude title compound (7.6 g, 20.0 mmol, 114%). The crude residue continued to be used in the next step without further purification.

¹H NMR (400 MHz, CDCl₃) δ7.33(s, 1H), 7.25(d, J = 7.6 Hz, 2H), 7.02(d, 8.1 Hz 1H), 6.07(s, 1H), 4.69(m, 2H), 3.27(m, 2H), 1.85(m, 1H), 0.93(m, 2H), 0.66(m, 2H); [M+H]⁺ 280.68

### Step 7: 4-Bromo-7-chloro-6-(4-cyclopropylbenzyl)-2,3-dihydrobenzofuran (Compound 40)

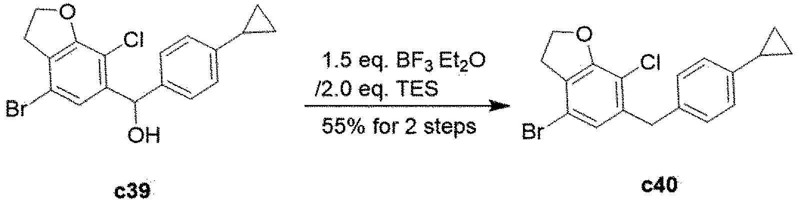

Triethylsilane (4.6 mL, 40 mmol) and BF₃OEt₂ (3.8 mL, 30 mmol) were added to a solution of 4-bromo-7-chloro-2,3-dihydrobenzofuran-6-yl)(4-cyclopropylphenyl)methanol (Compound 39) (7.6 g, 20 mmol) in DCM/acetonitrile (100 mL/100 mL) at -20°C under a nitrogen atmosphere. The mixture was gradually warmed to room temperature and stirred at room temperature for another 50 minutes. A saturated NaHCO₃ solution (200 mL) was slowly added to the reaction mixture to terminate the reaction, and the reaction mixture was extracted with ethyl acetate (100 mL). The organic layer was dried over MgSO₄, filtered, and concentrated under vacuum. The residue was purified by silica gel column chromatography to obtain the title compound (4.4 g, 12.1 mmol, 85% for two steps).

¹H NMR (400 MHz, CDCl₃) δ7.07(d, J = 8.0 Hz, 2H), 6.99(d, J = 8.0 Hz, 2H), 6.80(s, 1H), 4.70(t, J = 8.8 Hz, 2H), 3.97(s, 2H), 3.26(t, J = 8.8 Hz, 2H), 1.88-1.84(m, 1H), 0.95-0.90(m, 2H), 0.68-0.64(m, 2H): [M+H]⁺ 364.68

### Example 3: Synthesis of (2S,3R,4R,5S,6R)-2-(7-chloro-6-(4-cyclopropylbenzyl)-2,3-dihydrobenzofuran-4-yl)-6-(hydroxyymethyl)tetrahydro-2H-pyran-3,4,5-triol (c28)

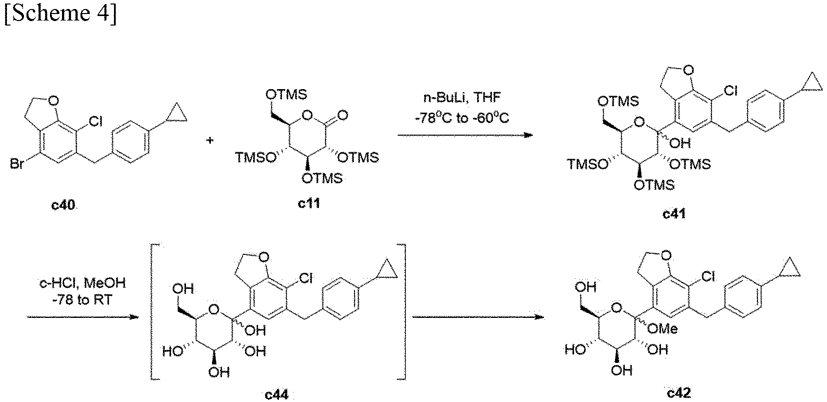

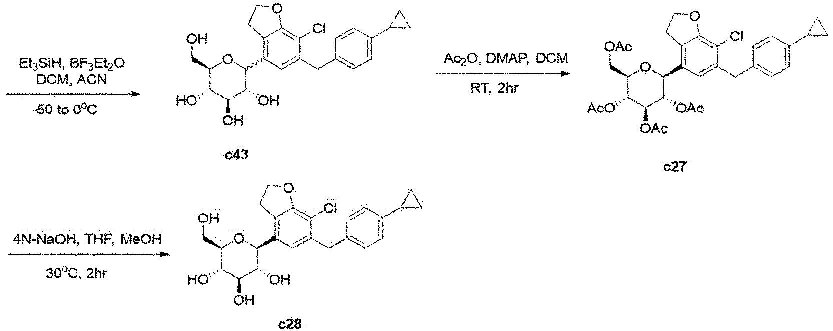

### Step 8: (3R,4S,SS,6R)-2-(7-chloro-6-(4-cyclopropylbenzyl)-2,3-dihydrobenzofuran-4-yl)-6-(hydroxymethyl)-2-methoxytetrahydro-2H-pyran-3,4,5-triol (Compound 42)

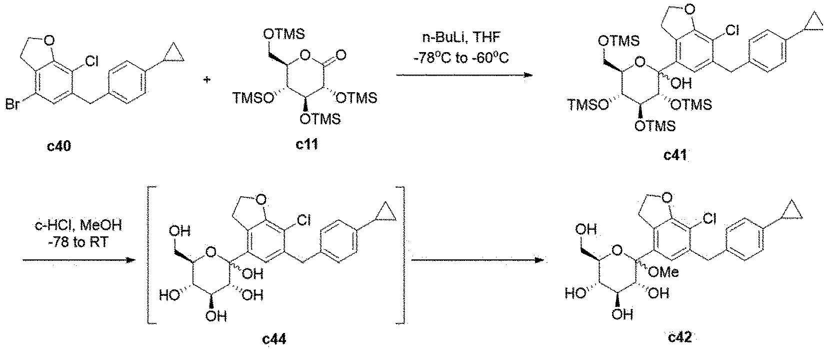

*n*-Butyllithium (2.5 M in hexane, 23.1 mL, 57.8 mmol) was added dropwise to a solution of 4-bromo-7-chloro-6-(4-cyclopropylbenzyl)-2,3-dihydrobenzofuran (10.0 g, 27.5 mmol) in THF (80 mL) at -78°C under a nitrogen atmosphere and stirred at the same temperature for 10 to 30 minutes. After the completion of the C3 production reaction was confirmed through TLC, a solution prepared by adding c-HCl (6.32 mL, 71.5 mmol) to MeOH (100 mL) was added to the reaction mixture to terminate the coupling reaction. At the same time, the TMS protecting group was removed to form compound c4 as an intermediate. To synthesize c5, the reaction temperature was gradually raised to room temperature, and the mixture was stirred for 7 hours or more. After the completion of the reaction was confirmed through TLC, an aqueous 3% NaHCO₃ solution (220 mL) was added and stirred for 10 minutes to terminate the reaction. After the reaction was completed, the solution was concentrated to 160 mL under reduced pressure. Ethyl acetate (60 mL) and 100 mL of an aqueous 20% NaCl solution were added to the concentrated reaction solution, stirred for 10 minutes, and then left to stand to separate an organic layer. The aqueous layer was re-extracted by adding ethyl acetate in an amount that is 6-fold the amount of the aqueous layer. After adding MgSO₄ (40 g) was added to the combined ethyl acetate layer to remove moisture, the layer was filtered and washed with ethyl acetate (20 mL). The filtrate was concentrated under reduced pressure to obtain the title compound c42, which was used in the next step without further purification.

[M+Na]+ 499 and [M-OMe]+ 445.

### Step 9: (2S,3R,4R,5S,6R)-2-(7-chloro-6-(4-cyclopropylbenzyl)-2,3-dihydrobenzofuran-4-yl)-6-(hydroxymethyl)tetrahydro-2H-pyran-3,4,5-triol (Compound c43)

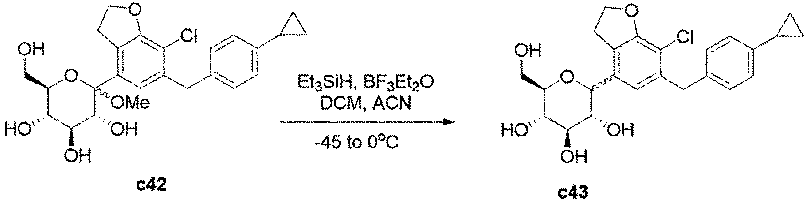

DCM (100 mL) and acetonitrile (100 mL) were added to the concentrated (3R,4S,5S,6R)-2-(7-chloro-6-(4-cyclopropylbenzyl)-2,3-dihydrobenzofuran-4-yl)-6-(hydroxymethyl)-2-methoxytetrahydro-2H-pyran-3,4,5-triol (Compound c42: 13.12 g, 27.5 mmol, 1.0 eq) under a nitrogen atmosphere, dissolved, and then cooled to -45°C. Et₃SiH (13.2 mL, 82.5 mmol) and BF₃Et₂O (10.2 mL, 82.5 mmol) were sequentially added to the cooled solution. The reaction mixture was stirred for 5 hours while gradually increasing the temperature from -45°C to 0°C. After the completion of the reaction was confirmed through TLC, an aqueous saturated NaHCO₃ solution (200 mL) was added to the reaction solution to terminate the reaction, and the mixture was concentrated to 220 mL. Ethyl acetate (100 mL) was added to the concentrated residue to extract an aqueous layer. After the extracted aqueous layer was re-extracted by adding ethyl acetate (50 mL), the ethyl acetate layers were combined and filtered with MgSO₄ (40 g). The filtrate was concentrated under vacuum to obtain the light brown title compound (12.29 g, 100%). The product was used directly in the next step without further purification.

### Step 10: (2R,3R,4R,5S,6S)-2-(acetoxymethyl)-6-(7-chloro-6-(4-cyclopropylbenzyl)-2,3-dihydrobenzofuran-4-yl)tetrahydro-2H-pyran-3,4,5-triyl triacetate (Compound 27)

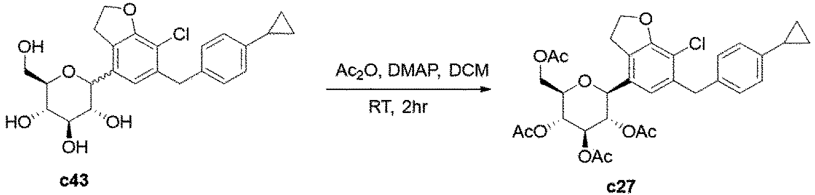

DCM (150 mL) was added to the concentrated Compound 43 (12.29 g, 27.5 mmol) and dissolved at room temperature in a nitrogen (gas) atmosphere. Thereafter, DMAP (4.04 g, 33.2 mmol) and acetic anhydride (720.8 mL, 220.8 mmol) were added thereto and stirred at room temperature for 2 hours. After the completion of the reaction was confirmed through TLC, 1 N HCl (100 mL) was added and stirred for 10 minutes to terminate the reaction. Then, the reaction solution was left to stand to separate the layers. After the aqueous layer was re-extracted by adding DCM (50 mL), the DCM layers were combined. MgSO₄ (20 g) was added thereto, and the combined DCM layers were filtered and dried. After MeOH (20 mL) was added to the filtrate, the filtrate was concentrated under vacuum. MeOH (100 mL) was added to the concentrate and stirred at room temperature for an hour to precipitate crystals, which were then filtered. The filtrate was washed with MeOH (20 mL) and then dried under vacuum at 50°C to obtain the white title compound (14.01 g, 82.9%).

¹H NMR (500 MHz, CDCl₃): δ7.04-7.02(m, 2H), 6.98-6.95(m, 2H), 6.53(s, 2H), 5.29-5.24(m, 1H), 5.18-5.12(m, 2H), 4.71-4.65(m, 2H), 4.31-4.26(m, 1H), 4.25-4.22(m, 1H), 4.15-4.11(m, 1H), 4.15-4.11(m, 1H), 4.05-3.91(m, 2H), 3.79-3.74(m, 1H), 3.40-3.35(m, 2H), 2.60(s, 3H), 2.05(s, 3H), 1.99(s, 3H), 1.88-1.81(m, 1H), 1.66(s, 3H), 0.94-0.89(m, 2H), 0.66-0.61(m, 2H); [M+Na]+ 637.

### Step 11: (2S,3R,4R,5S,6R)-2-(7-chloro-6-(4-cyclopropylbenzyl)-2,3-dihydrobenzofuran-4-yl)-6-(hydroxymethyl)tetrahydro-2H-pyran-3,4,5-triol (Compound 28)

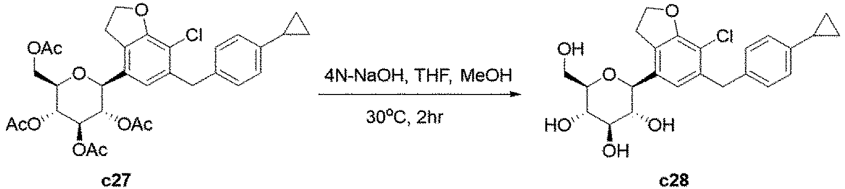

THF (25 mL) and methanol (25 mL) were added to Compound 27 (5.0 g, 8.13 mmol). After the mixture was heated to 30°C to 35°C, a 4 N NaOH solution (10.2 mL, 40.7 mmol) was added to the slurry-phase solution and stirred for 2 hours. After the completion of the reaction was confirmed through TLC, the reaction solution was cooled to 0°C, and 1 N HCl was then added to adjust the pH of the reaction solution to 6.8 to 7.0 in order to terminate the reaction. The reaction mixture was concentrated under reduced pressure to 60 mL and extracted by adding water (100 mL) and ethyl acetate (100 mL). After the aqueous layer was re-extracted by adding ethyl acetate (100 mL). MgSO₄ (20 g) was then added thereto, and the aqueous layer was dried, filtered, and then concentrated under reduced pressure. The concentrated residue was thoroughly dissolved at 80°C by adding ethyl acetate (55 mL) and then slowly cooled to room temperature to precipitate crystals, which were then stirred for an hour. IPE (91 mL) was added dropwise to the crystallization solution over 30 minutes, cooled to 0°C, and then stirred at 0°C to 5°C for another one hour. The solution was left to stand at 0°C to 5°C for an hour, filtered, washed twice with a 1:1 IPE:ethyl acetate solution, and then dried under vacuum at 50°C to obtain the title compound as a white solid (3.4 g, 93.1%).

¹H NMR (500 MHz, CDCl₃): δ7.02(d, J = 8.0 Hz, 2H), 6.92(d, J = 8.0 Hz, 2H), 6.81(s, 1H), 4.59(t, J = 8.8 Hz, 2H), 4.11(d, J = 9.2 Hz, 1H), 3.96(19.0 Hz, 15.2 Hz, 2H), 3.87-3.84(m, 1H), 3.67-3.63(m, 1H), 3.47-3.37(m, 3H), 3.35-3.33(m, 3H), 1.85-1.79(m, 1H), 0.91-0.86(m, 2H), 0.61-0.57(m, 2H); [M+Na]+ 469

### Example 4: Synthesis of 4-bromo-7-chloro-2,3-dihydrobenzofuran-6-carboxylic acid (c51)

Scheme 5 below schematically shows a process of synthesizing compound c40 exemplified in Example 4.

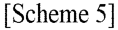

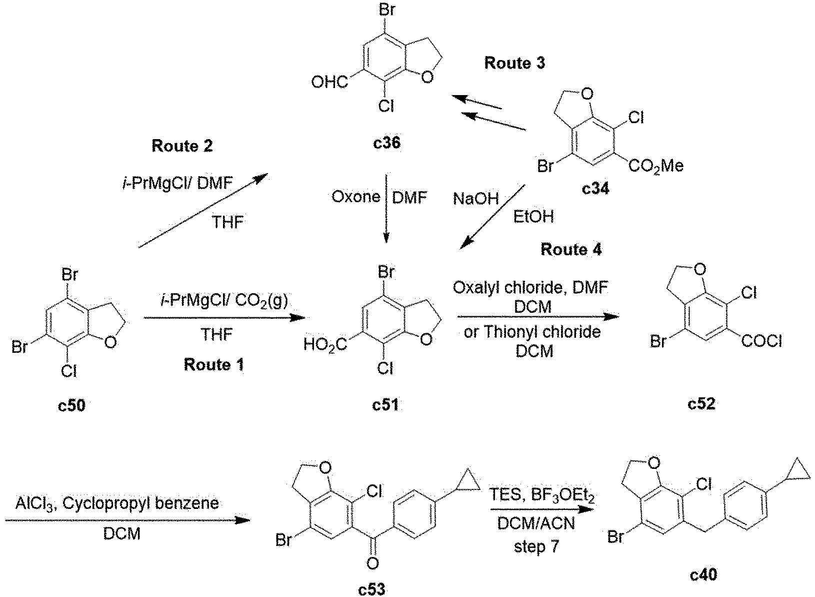

### [Route 1] Synthesis of 4-bromo-7-chloro-2,3-dihydrobenzofuran-6-carboxylic acid (c51)

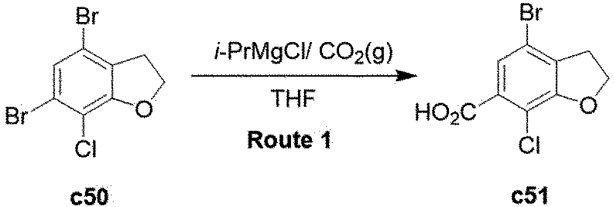

### Step 1: 4-Bromo-7-chloro-2,3-dihydrobenzofuran-6-carboxylic acid (Compound c51)

4,6-Dibromo-7-chloro-2,3-dihydrobenzofuran (c50, 2.0 g, 6.40 mmol) was added to a reactor under a nitrogen atmosphere and dissolved by adding THF (20 mL). The reactor was cooled to 0°C to 5°C, and isopropylmagnesium chloride (1 M in THF, 9.6 mL, 9.60 mmol) was added dropwise. After the mixture was stirred at the same temperature for 15 minutes, the temperature of the mixture was raised to room temperature, and stirring was performed for another 15 minutes. A cannula was connected to the reaction solution, and CO₂ (g) was added for an hour. After the completion of the reaction was confirmed through TLC, the reaction solution was cooled to 0°C to 5°C. 1 N HCl (20 mL) was added to the reactor to terminate the reaction, and then extraction was performed twice with ethyl acetate (20 mL). Na₂SO₄ was added to the combined ethyl acetate layers to remove moisture. Then, the ethyl acetate layer was filtered, and concentrated under vacuum. Thereafter, methyl *t*-butyl ether (4 mL) was added to the concentrate and stirred at room temperature. Hexane (12 mL) was additionally added thereto, and the crystals were aged for an hour. The resulting crystals were filtered, washed with hexane (4 mL), and dried under vacuum at 50°C for 12 hours to obtain 1.42 g (80%) of the title compound c51.

¹H NMR (500 MHz, CDCl₃): δ7.69 (s, 1H), 4.76 (t, J = 9.0 Hz, 2H), 3.35 (t, J = 9.0 Hz, 2H); LC-MS: [M+H]+ 277.

### [Route 2] Synthesis of 4-bromo-7-chloro-2,3-dihydrobenzofuran-6-carboxylic acid (c51)

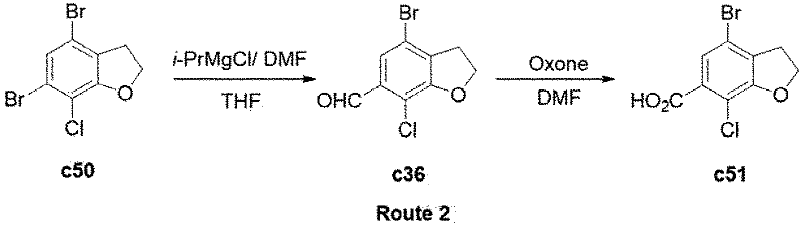

### Step 1: 4-Bromo-7-chloro-2,3-dihydrobenzofuran-6-carbaldehyde (c36)

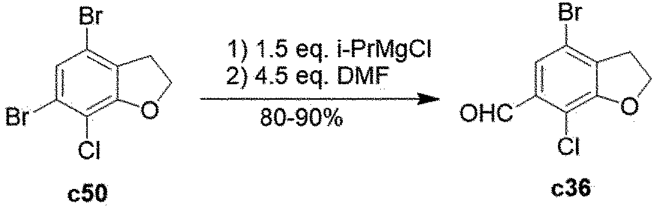

THF (22.5 L) was added to a reactor, and 4,6-dibromo-7-chloro-2,3-dihydrobenzofuran (c50: 2.25 kg, 1.0 eq) was added and dissolved. The air was replaced three times with nitrogen, and the solution was then cooled to 0°C to 5°C. Isopropylmagnesium chloride (5.1 kg, 5.6 L, 2M in THF, 1.5 eq.) was added dropwise to the solution and then stirred for 30 minutes. The temperature of the reaction solution was raised again to 25°C to 30°C, and DMF (2.4 kg, 4.5 eq) was added and stirred at 25°C to 30°C for 30 minutes. After the completion of the reaction was confirmed, the reaction solution was cooled to 0°C to 10°C. Acetic acid (2.1 L) was added to adjust the pH to 4 to 6, and water (22.5 L) was then added to complete the reaction. After the reaction was completed, the solution was concentrated under vacuum to completely remove THF, cooled again to 0 to 10°C, and then filtered. The filtrate was washed with water (6.7 L). The resulting crystals were added to the reactor along with *n*-heptane (17 L), heated to 90°C, and stirred for 30 minutes. The solution was cooled again to 0°C to 10°C and stirred for 2 hours. Then, the crystals were filtered and washed with n-heptane (4.5 L). The obtained crystals were dried under vacuum at 50°C to 60°C for 16 hours to obtain the yellow title compound (1.6 kg, 85%).

¹H NMR (500 MHz, CDCl₃) δ10.34(s, 1H), 7.60(s, 1H), 4.80(t, J = 9.2 Hz, 1H), 3.35(t, J = 9.2 Hz, 1H); [M+H]+ 263

### Step 2: 4-Bromo-7-chloro-2,3-dihydrobenzofuran-6-carboxylic acid (c51)

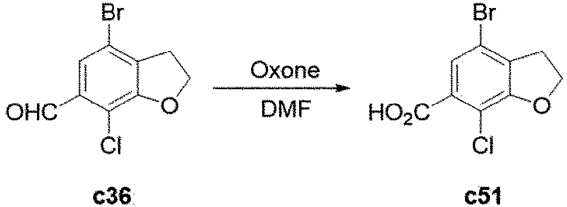

After DMF (300 mL) was added to a reactor, 4-bromo-7-chloro-2,3-dihydrobenzofuran-6-carbaldehyde (c36, 60 g, 229.5 mmol) was added and thoroughly dissolved at room temperature. Oxone (48.9 g, 321.3 mmol) was added in portions at 20°C to 30°C and stirred for 6 hours. After the completion of the reaction was confirmed through TLC, methyl t-butyl ether (300 mL) was added to precipitate crystals. The resulting crystals were filtered and washed with methyl t-butyl ether (120 mL). Water (600 mL) was added to the filtrate and stirred for 10 minutes to separate the layers. Thereafter, the layers were additionally extracted twice with methyl t-butyl ether (180 mL). The combined organic layers were washed twice with water (180 mL) and then concentrated under reduced pressure. Methyl t-butyl ether (120 mL) was added to the concentrate and stirred for 10 minutes. Then, hexane (360 mL) was additionally added, and the crystals were aged for an hour. The resulting crystals were filtered, washed with hexane (60 mL), and dried under vacuum at 50°C for 12 hours to obtain 57.3 g (90.0%) of the title compound c51.

¹H NMR (500 MHz, CDCl₃): δ7.69 (s, 1H), 4.76 (t, J = 9.0 Hz, 2H), 3.35 (t, J = 9.0 Hz, 2H); LC-MS: [M+H]+ 277.

### [Route 3] Synthesis of 4-bromo-7-chloro-2,3-dihydrobenzofuran-6-carboxylic acid (c51)

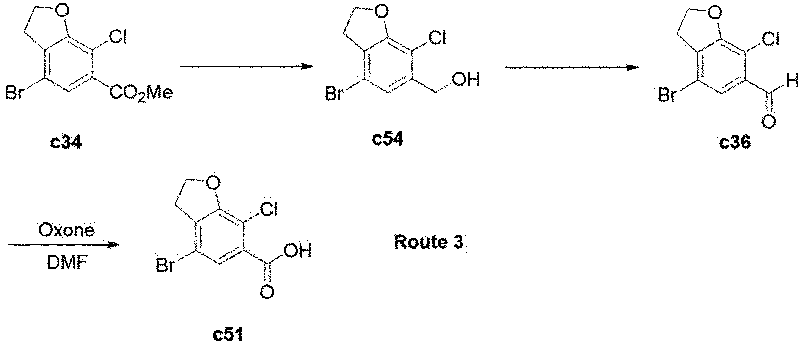

### Step 1: (4-Bromo-7-chloro-2,3-dihydrobenzofuran-6-yl)methanol (c54)

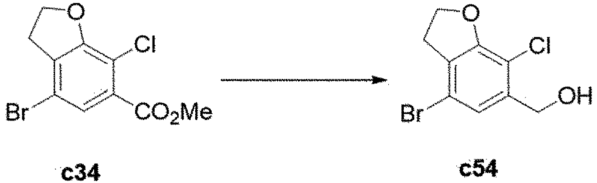

Sodium borohydride (5.07 g, 133.98 mmol) was slowly added to a mixture of methyl 4-bromo-7-chloro-2,3-dihydrobenzofuran-6-carboxylate (c34, 13.0 g, 44.7 mmol) in THF/EtOH (150 mL/75 mL) at room temperature. The mixture was stirred at room temperature for 12 hours. Saturated NH₄Cl (0°C) was added to the resulting mixture to terminate the reaction, and the mixture was then extracted with EtOAc (neutral pH 7.0). The organic layer was dried over MgSO₄, filtered, and concentrated under vacuum to obtain the title compound (11.7 g, 99%) as a white solid. The crude product was used in the next step without further purification.

¹H NMR (500 MHz, CDCl₃) δ7.15(s, 1H), 4.73(m, 4H), 3.29(t, J = 8.8 Hz, 2H), 1.91(t, J = 6.4 Hz, 1H); [M-H₂O]+ 245.

### Step 2: 4-Bromo-7-chloro-2,3-dihydrobenzofuran-6-carbaldehyde (c36)

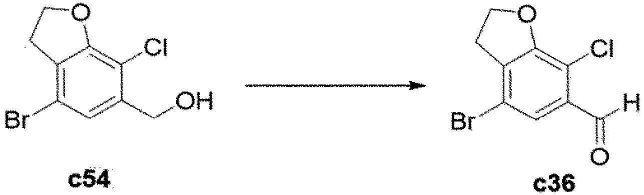

Pyridinium chlorochromate (14.4 g, 66.6 mmol) was slowly added to a solution of (4-bromo-7-chloro-2,3-dihydrobenzofuran-6-yl)methanol (11.7 g, 44.4 mmol) in CH₂Cl₂ (450 mL) at room temperature. After the mixture was stirred for 8 hours, the precipitate was filtered using a silica gel pad and washed with CH₂Cl₂. The filtrate was concentrated under vacuum to give the title compound (10.4 g, 39.8 mmol, 90%) as a white solid. The crude product was used in the next step without further purification.

¹H NMR (500 MHz, CDCl₃) δ10.33(s, 1H), 7.59(s, 1H), 4.79(t, J = 8.8 Hz, 2H), 3.35(t, J = 8.8 Hz, 2H); [M+H]+ 261.

### [Route 4] Synthesis of 4-bromo-7-chloro-2,3-dihydrobenzofuran-6-carboxylic acid (c51)

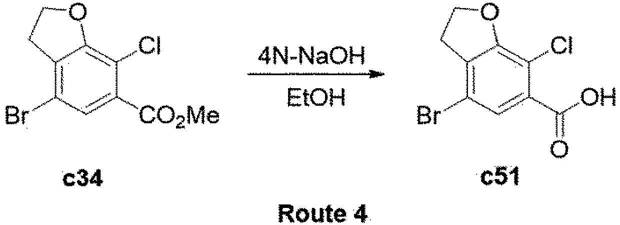

### Step 1: 4-Bromo-7-chloro-2,3-dihydrobenzofuran-6-carboxylic acid (c51)

4 N NaOH (51.4 mL, 205.8 mmol) was added to a mixture of methyl 4-bromo-7-chloro-2,3-dihydrobenzofuran-6-carboxylate (c34, 20.0 g, 68.6 mmol) in ethanol (200 mL) at room temperature. The mixture was stirred at room temperature for 2 hours, and the completion of the reaction was confirmed through TLC. Thereafter, 1 N HCl was added to the reaction solution (acidic pH 1.0) to terminate the reaction, and the reaction solution was extracted with ethyl acetate. The organic layer was dried over anhydrous magnesium sulfate, filtered, and concentrated under vacuum to obtain the title compound c51 (18.3 g, 44.4 mmol, 96.3%) as a white solid.

¹H NMR (500 MHz, CDCl₃): δ7.69 (s, 1H), 4.76 (t, J = 9.0 Hz, 2H), 3.35 (t, J = 9.0 Hz, 2H); LC-MS: [M+H]+ 277.

### Example 5: Synthesis of 4-bromo-7-chloro-6-(4-cyclopropylbenzyl)-2,3-dihydrobenzofuran (c40)

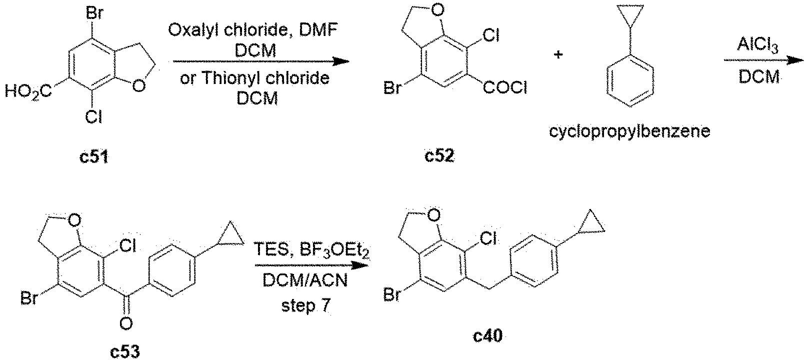

### Step 1-1: Synthesis of 4-bromo-7-chloro-2,3-dihydrobenzofuran-6-carbonyl chloride (c52)

c51 (5.0 g, 18.1 mmol) was dissolved in DCM (30 mL) under a nitrogen atmosphere, and DMF (0.5% by weight) was added as a catalyst. The reaction solution was cooled to 0°C to 10°C, and then oxalyl chloride (1.87 mL, 21.8 mmol, 1.2 eq) was added dropwise thereto. The reaction solution was heated to 20°C to 30°C and stirred for an hour. After the completion of the reaction was confirmed, the reaction solution was concentrated under vacuum. DCM (10 mL) was added again to the concentrate, and then concentration under vacuum was performed to remove excess oxalyl chloride, and the quantitatively obtained off-white target compound was directly used in the next reaction without purification.

### Step 1-2: Synthesis of 4-bromo-7-chloro-2,3-dihydrobenzofuran-6-carbonyl chloride (c52)

c51 (5.0 g, 18.1 mmol) was dissolved in DCM (30 mL) under a nitrogen atmosphere, and thionyl chloride (2.63 mL, 36.2 mmol, 2.0 eq) was added dropwise at 20°C to 30°C. After the addition was completed, the mixture was heated and stirred under reflux for 2 hours. After the completion of the reaction was confirmed, the reaction solution was concentrated under vacuum. DCM (10 mL) was added again to the concentrate, and then concentration under vacuum was performed to remove excess thionyl chloride, and the quantitatively obtained off-white target compound was directly used in the next reaction without purification.

### Step 2: Synthesis of (4-bromo-7-chloro-2,3-dihydrobenzofuran-6-yl)(4-cyclopropylphenyl)methanone (c53)

c51 (1.00 g, 3.60 mmol) was dissolved in dichloromethane (30 mL) at room temperature under a nitrogen atmosphere, and DMF (0.01 mL, 0.13 mmol) was added, followed by dropwise addition of oxalyl chloride (0.34 mL, 3.96 mmol). The mixture was stirred at room temperature for an hour and then cooled to -15°C. Thereafter, cyclopropyl benzene (0.91 mL, 7.20 mmol) was added to the reaction mixture and stirred for 5 minutes, and AlCl₃ (0.58 g, 4.32 mmol) was added to the reaction mixture and stirred at the same temperature for 60 minutes. After the completion of the reaction was confirmed through TLC, an aqueous 1 N HCl solution was added to the reaction solution to terminate the reaction, and the reaction solution was extracted with ethyl acetate. The organic layer obtained by extraction was dried over anhydrous magnesium sulfate, filtered, and then concentrated under vacuum. The concentrated residue was purified by silica gel chromatography to obtain the title compound c40 (1.18 g, 86.7%) as a white solid.

¹H NMR (500 MHz, CDCl₃): δ7.70 (d, *J =* 8.0 Hz, 2H), 7.11 (d, *J =* 8.0 Hz, 2H), 6.99 (s, 1H),4.78 (t, *J =* 9.0 Hz, 2H), 3.36 (t, *J =* 7.2 Hz, 2H), 1.97-1.94 (m, 1H), 1.10-1.07 (m, 2H), 0.82-0.81 (m, 2H) ; LC-MS: [M+H]⁺ 377.

### Step 3: Synthesis of 4-bromo-7-chloro-6-(4-cyclopropylbenzyl)-2,3-dihydrobenzofuran (c40)

In a reactor, dichloromethane (10 mL) and acetonitrile (10 mL) were added to (4-bromo-7-chloro-2,3-dihydrobenzofuran-6-yl)(4-cyclopropylphenyl)methanone (c53) (0.97 g, 2.57 mmol), dissolved, and then cooled to -15°C. EtsSiH (1.2 mL, 7.71 mmol) and BF₃-Et₂O (0.79 mL, 6.42 mmol) were sequentially added to the reaction solution, and the reaction mixture was then warmed to room temperature and stirred for 4 hours. After the completion of the reaction was confirmed through TLC, an aqueous saturated NaHCO₃ solution (40 mL) was added to terminate the reaction, and the reaction solution was extracted with ethyl acetate. The organic layer obtained by extraction was dried over anhydrous magnesium sulfate, filtered, and concentrated under vacuum. The concentrated residue was purified by silica gel chromatography to obtain the target compound c40 (0.84 g, 89.9%) as a white solid.

¹H NMR (500 MHz, CDCl₃): δ7.07 (d, J = 10.0 Hz, 2H), 6.99 (d, J = 10.0 Hz, 2H), 6.80 (s, 1H), 4.70 (t, J = 11.0 Hz, 2H), 3.97 (s, 2H), 3.26 (t, J = 11.0 Hz, 2H), 1.88-1.84 (m, 1H), 0.95-0.90 (m, 2H), 0.68-0.64 (m, 2H); LC-MS: [M+H]+ 363.

**[Table 4]**

| | 2017-0142904 | Modified process (Scheme 3) | Modified process (Scheme 5) |
|---|---|---|---|
| Number of c40 production steps | 14 steps | 7 steps | 7 to 8 steps |
| Special equipment | Ozone reactor | Not required | Not required |
| Coupling compound | 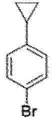 | 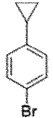 | 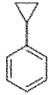 |
| Total yield | 26% | 39% | 36% |
| c40 purity | 99.0% or more | 99.0% or more | 99.0% or more |
| Individual related materials of c40 | 0.2% or less | 0.2% or less | 0.2% or less |
| C28 purity | 99.0% or more | 99.0% or more | 99.0% or more |
| Individual related materials of C28 | 0.1 or less | 0.1 or less | 0.1 or less |

Scheme 3 can secure competitiveness over the existing c40 synthesis method of Korean Patent Publication No. 2017-0142904 because it has a fewer number of reaction steps, and Scheme 5 utilizes the c50 intermediate to secure competitiveness by optimizing Friedel Crafts acylation reaction conditions using cyclopropyl benzene, which is 25% cheaper and has easier quality control compared to expensive 1-bromo-4-cyclopropylbenzene, as a coupling compound.

## Claims

1. A method for producing a compound of Chemical Formula 9, comprising the following steps:
obtaining a compound of Chemical Formula 6 from a compound of Chemical Formula 5; and
reacting a compound of Chemical Formula 7 with the compound of Chemical Formula 6 to obtain a compound of Chemical Formula 8: 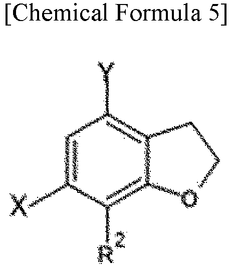 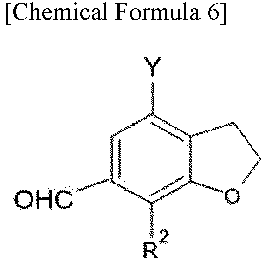 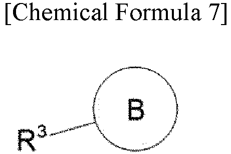 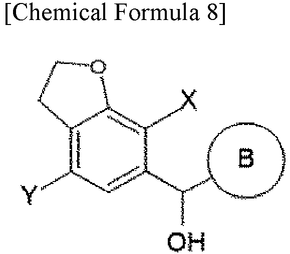 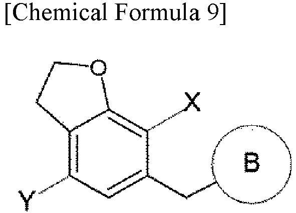 wherein:
R², R³, X, and Y are each independently a halogen, and
B is 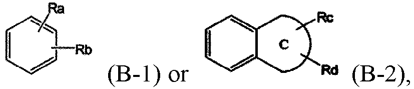
wherein Ra, Rb, Rc, and Rd are each independently hydrogen, a halogen, hydroxy, mercapto, cyano, nitro, amino, carboxy, oxo, a C1-7 alkyl, a C1-7 alkylthio, a C2-7 alkenyl, a C2-7 alkynyl, a C1-7 alkoxy, a C1-7 alkoxy-C1-7 alkyl, a C2-7 alkenyl-C1-7 alkyloxy, a C2-7 alkynyl-C1-7 alkyloxy, a C3-10 cycloalkyl, a C3-7 cycloalkylthio, a C5-10 cycloalkenyl, a C3-10 cycloalkyloxy, a C3-10 cycloalkyloxy-C1-7 alkoxy, a phenyl-C1-7 alkyl, a C1-7 alkylthio-phenyl, a phenyl-C1-7 alkoxy, a mono- or di-C1-7 alkylamino, a mono- or di-C1-7 alkylamino-C1-7 alkyl, a C1-7 alkanoyl, a C1-7 alkanoylamino, a C1-7 alkylcarbonyl, a C1-7 alkoxycarbonyl, carbamoyl, a mono- or di-C1-7 alkylcarbamoyl, a C1-7 alkylsulfonylamino, phenylsulfonylamino, a C1-7 alkylsulfinyl, a C6-14 arylsulfanyl, a C6-14 arylsulfonyl, a C6-14 aryl, a 5-13-membered heteroaryl, a 5-10-membered heterocycloalkyl, a 5-10-membered heterocycloalkyl-C1-7 alkyl, or a 5-10-membered heterocycloalkyl-C1-7 alkoxy;
ring C is a C3-10 cycloalkyl, a C5-10 cycloalkenyl, a C6-14 aryl, a 5-13-membered heteroaryl, or a 5-10-membered heterocycloalkyl;
the alkyl, the alkenyl, the alkynyl, and the alkoxy are each independently unsubstituted or have one or more substituents selected from the group consisting of a halogen, hydroxy, cyano, nitro, amino, mercapto, a C1-7 alkyl, and a C2-7 alkynyl;
the cycloalkyl, the cycloalkenyl, the aryl, the heteroaryl, and the heterocycloalkyl are each independently unsubstituted or have one or more substituents selected from the group consisting of a halogen, hydroxy, cyano, nitro, amino, mercapto, a C1-4 alkyl, and a C1-4 alkoxy; and
the heteroaryl and the heterocycloalkyl each independently contain one or more heteroatoms selected from the group consisting of N, S, and O.

2. The method of claim 1, wherein the obtaining of the compound of Chemical Formula 6 from the compound of Chemical Formula 5 comprises:
selectively converting the substituent X in the compound of Chemical Formula 5 to an aldehyde group using an organomagnesium reagent and dimethylformamide (DMF) as reaction reagents.

3. The method of claim 2, wherein the organomagnesium reagent is used in an amount of 1 to 2 equivalents relative to one equivalent of the compound of Chemical Formula 5, and the dimethylformamide is used in an amount of 3 to 6 equivalents relative to one equivalent of the compound of Chemical Formula 5.

4. The method of claim 2, wherein the reaction is performed using tetrahydrofuran (THF), diethyl ether, 2-methyl tetrahydrofuran, and a mixture thereof as a reaction solvent, and the reaction solvent is used in an amount that is 5- to 15-fold (w/v) the amount of the compound of Chemical Formula 5.

5. The method of claim 2, wherein the reaction is performed at -10°C to 50°C.

6. The method of claim 1, wherein the reacting of the compound of Chemical Formula 7 with the compound of Chemical Formula 6 to obtain the compound of Chemical Formula 8 comprises:
lithiating a halogen position of the compound of Chemical Formula 7 and then coupling the same with the compound of Chemical Formula 6.

7. The method of claim 6, wherein the lithiation is performed using 1 to 1.3 equivalents of an organolithium reagent relative to one equivalent of the compound of Chemical Formula 7.

8. The method of claim 6, further comprising:
adding butylmagnesium chloride or *tert*-butylmagnesium chloride to the compound of Chemical Formula 7 in a solvent prior to the lithiation reaction.

9. The method of claim 8, wherein the butylmagnesium chloride or *tert-*butylmagnesium chloride is used in an amount of 0.2 to 1 equivalent relative to one equivalent of the compound of Chemical Formula 7.

10. The method of claim 1, wherein the reacting of the compound of Chemical Formula 7 with the compound of Chemical Formula 6 to obtain the compound of Chemical Formula 8 comprises:
obtaining a compound of Chemical Formula 7' from the compound of Chemical Formula 7; and
coupling the compound of Chemical Formula 7' with the compound of Chemical Formula 6,

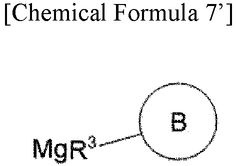

11. The method of claim 1, wherein the compound of Chemical Formula 5 is obtained by halogenating the amine in a compound of Chemical Formula 4 through the Sandmeyer reaction: 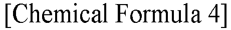 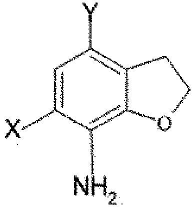 wherein:
X and Y are each independently a halogen.

12. The method of claim 11, wherein the Sandmeyer reaction includes reacting the compound of Chemical Formula 4 with NaNO₂ to form a diazonium salt of the compound of Chemical Formula 4 and reacting the diazonium salt with a transition metal salt to obtain the compound of Chemical Formula 5.

13. The method of claim 12, wherein the NaNO₂ is used in an amount of 1 to 2 equivalents relative to the compound of Chemical Formula 4, and the reaction of the transition metal salt with the diazonium salt of the compound of Chemical Formula 4 is performed at 30°C to 80°C.

14. A method for producing a compound of Chemical Formula 9, comprising the following steps:
obtaining a compound of Chemical Formula 11 from a compound of Chemical Formula 10;
reacting a compound of Chemical Formula 12 with the compound of Chemical Formula 11 to obtain a compound of Chemical Formula 13; and
obtaining the compound of Chemical Formula 9 from the compound of Chemical Formula 13: 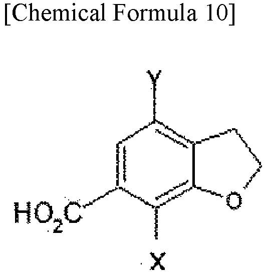 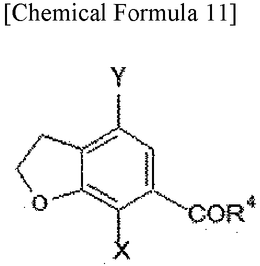 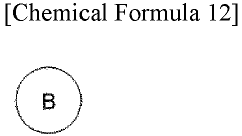 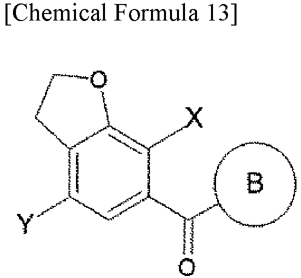 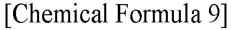 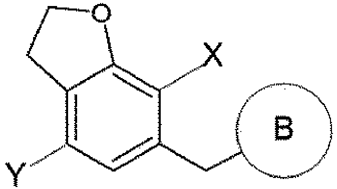 wherein:
X, Y, and R⁴ are each independently a halogen, and
B is 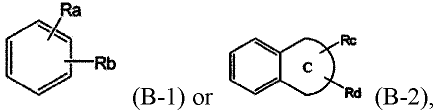
wherein Ra, Rb, Rc, and Rd are each independently hydrogen, a halogen, hydroxy, mercapto, cyano, nitro, amino, carboxy, oxo, a C1-7 alkyl, a C1-7 alkylthio, a C2-7 alkenyl, a C2-7 alkynyl, a C1-7 alkoxy, a C1-7 alkoxy-C1-7 alkyl, a C2-7 alkenyl-C1-7 alkyloxy, a C2-7 alkynyl-C1-7 alkyloxy, a C3-10 cycloalkyl, a C3-7 cycloalkylthio, a C5-10 cycloalkenyl, a C3-10 cycloalkyloxy, a C3-10 cycloalkyloxy-C1-7 alkoxy, a phenyl-C1-7 alkyl, a C1-7 alkylthio-phenyl, a phenyl-C1-7 alkoxy, a mono- or di-C1-7 alkylamino, a mono- or di-C1-7 alkylamino-C1-7 alkyl, a C1-7 alkanoyl, a C1-7 alkanoylamino, a C1-7 alkylcarbonyl, a C1-7 alkoxycarbonyl, carbamoyl, a mono- or di-C1-7 alkylcarbamoyl, a C1-7 alkylsulfonylamino, phenylsulfonylamino, a C1-7 alkylsulfinyl, a C6-14 arylsulfanyl, a C6-14 arylsulfonyl, a C6-14 aryl, a 5-13-membered heteroaryl, a 5-10-membered heterocycloalkyl, a 5-10-membered heterocycloalkyl-C1-7 alkyl, or a 5-10-membered heterocycloalkyl-C1-7 alkoxy;
ring C is a C3-10 cycloalkyl, a C5-10 cycloalkenyl, a C6-14 aryl, a 5-13-membered heteroaryl, or a 5-10-membered heterocycloalkyl;
the alkyl, the alkenyl, the alkynyl, and the alkoxy are each independently unsubstituted or have one or more substituents selected from the group consisting of a halogen, hydroxy, cyano, nitro, amino, mercapto, a C1-7 alkyl, and a C2-7 alkynyl;
the cycloalkyl, the cycloalkenyl, the aryl, the heteroaryl, and the heterocycloalkyl are each independently unsubstituted or have one or more substituents selected from the group consisting of a halogen, hydroxy, cyano, nitro, amino, mercapto, a C1-4 alkyl, and a C1-4 alkoxy; and
the heteroaryl and the heterocycloalkyl each independently contain one or more heteroatoms selected from the group consisting of N, S, and O.

15. The method of claim 14, wherein the compound of Chemical Formula 10 is obtained by carboxylating a compound of Chemical Formula 5: 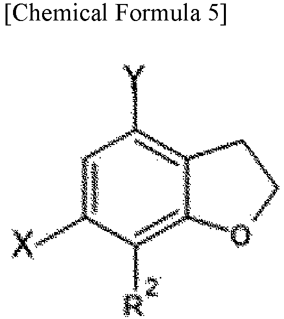 wherein:
R², X and Y are each independently a halogen.

16. The method of claim 14, wherein the compound of Chemical Formula 10 is obtained by converting the aldehyde group in a compound of Chemical Formula 6 to a carboxyl group: 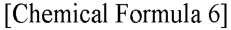 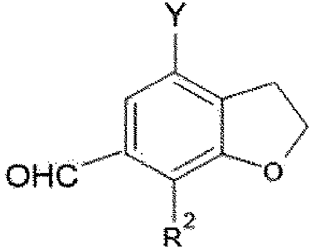 wherein:
R² and Y are each independently a halogen.

17. The method of claim 14, wherein the compound of Chemical Formula 10 is obtained by carboxylating a compound of Chemical Formula 14: 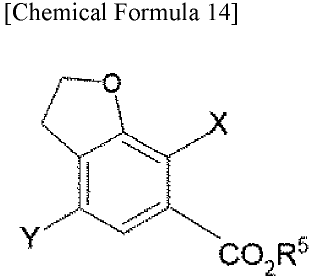 wherein:
X and Y are each independently a halogen, and
R⁵ is a C1-4 alkyl.

18. The method of claim 16, wherein the compound of Chemical Formula 6 is obtained by selectively converting the substituent X in a compound of Chemical Formula 5 to an aldehyde group: 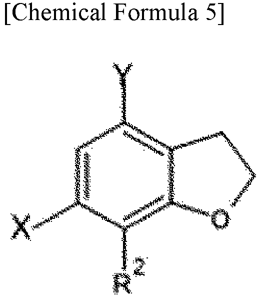 wherein:
R², X and Y are each independently a halogen.

19. The method of claim 16, wherein the compound of Chemical Formula 6 is obtained by:
obtaining a compound of Chemical Formula 15 from a compound of Chemical Formula 14, and
obtaining the compound of Chemical Formula 6 from the compound of Chemical Formula 15: 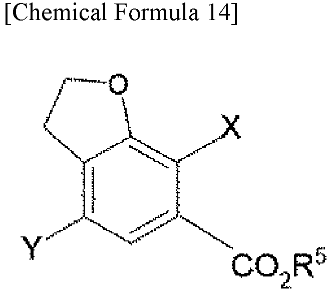 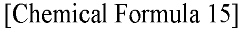 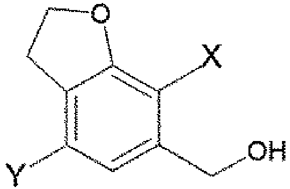 wherein:
X and Y are each independently a halogen, and
R⁵ is a C₁₋₄ alkyl.
